(19) **Europäisches Patentamt / European Patent Office / Office européen des brevets**

(11) **EP 4 631 514 A1**

(12) **EUROPEAN PATENT APPLICATION**
published in accordance with Art. 153(4) EPC

(43) Date of publication:
**15.10.2025 Bulletin 2025/42**

(21) Application number: **23900081.3**

(22) Date of filing: **08.12.2023**

(51) International Patent Classification (IPC):
**A61K 38/00** (2006.01)  **A61K 38/28** (2006.01)
**A61K 45/00** (2006.01)  **A61K 47/32** (2006.01)
**A61K 47/26** (2006.01)  **A61K 9/00** (2006.01)
**A61P 3/10** (2006.01)

(52) Cooperative Patent Classification (CPC):
**A61K 9/00; A61K 38/00; A61K 38/28; A61K 45/00;**
**A61K 47/26; A61K 47/32; A61P 3/10**

(86) International application number:
**PCT/CN2023/137373**

(87) International publication number:
**WO 2024/120513 (13.06.2024 Gazette 2024/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.12.2022 CN 202211570560**

(71) Applicant: **Hefei Tianhui Biotech Co., Ltd.
Hefei, Anhui 230601 (CN)**

(72) Inventors:
• **ZHANG, Lei**
  **Hefei, Anhui 230601 (CN)**
• **XU, Ping**
  **Hefei, Anhui 230601 (CN)**
• **CHEN, Shuhao**
  **Hefei, Anhui 230601 (CN)**
• **LV, Hui**
  **Hefei, Anhui 230601 (CN)**
• **PANG, Di**
  **Hefei, Anhui 230601 (CN)**
• **FAN, Aijia**
  **Hefei, Anhui 230601 (CN)**

(74) Representative: **Flesselles, Bruno F.G.
BF IP
36 rue Jean de la Fontaine
75016 Paris (FR)**

(54) **ORAL POLYPEPTIDE COMPOSITION AND USE THEREOF**

(57) An oral polypeptide composition comprising a polypeptide, fish oil, a protease inhibitor, at least one absorption enhancer, and at least one surfactant, and use thereof for treating a disease.

EP 4 631 514 A1

Description

FIELD OF THE INVENTION

The present invention belongs to the field of drug preparation technology, and specifically, relates to a polypeptide composition for oral administration and use thereof.

BACKGROUND OF THE INVENTION

During the treatment of a disease, polypeptide drugs have high specificity and effectiveness, making them an ideal choice for treating many human diseases. With the development of biotechnology, the clinical application of polypeptide drugs such as calcitonin, insulin and thymosin is becoming increasingly widespread. Nevertheless, the bioavailability of polypeptide drugs *in vivo* is low, and generally, they can be administered only by injection. However, side effects such as infection and inflammation are easily caused by injection administration, and patients have difficulty in overcoming fear of needles and have poor compliance, which reduces their motivation to take medication on time.
Oral administration is the most widely used and easily accepted method for drug delivery, but the oral absorption of polypeptide drugs is limited by the harsh environment of the gastrointestinal tract. Therefore, there is an urgent need in the art to provide a polypeptide drug delivery system that enables oral administration of polypeptide drugs.

SUMMARY OF THE INVENTION

An objective of the present invention is to provide a polypeptide composition for oral administration and use thereof so as to overcome shortcomings existing in the prior art. The oral polypeptide composition provided by the present invention can achieve oral administration of polypeptide drugs.
In one embodiment, the present invention provides an oral polypeptide composition, wherein the oral polypeptide composition comprises a polypeptide and fish oil.
In one embodiment, the present invention provides an oral polypeptide composition, wherein the oral polypeptide composition comprises a polypeptide and a protease inhibitor.
In one embodiment, the present invention provides an oral polypeptide composition, wherein the oral polypeptide composition comprises a polypeptide and an absorption enhancer.
In one embodiment, the present invention provides an oral polypeptide composition, wherein the oral polypeptide composition comprises a polypeptide, fish oil, a protease inhibitor, at least one absorption enhancer and at least one surfactant, in which

the polypeptide is present in an amount of 0.1 to 100 parts by weight;
the fish oil is present in an amount of 0.01 to 800 parts by weight;
the protease inhibitor is present in an amount of 0.1 to 210 parts by weight;
the absorption enhancer(s) is/are present in an amount of 0.1 to 320 parts by weight; and
the surfactant(s) is/are present in an amount of 1 to 800 parts by weight;

preferably,

the polypeptide is present in an amount of 5 to 50 parts by weight;
the fish oil is present in an amount of 30 to 400 parts by weight;
the protease inhibitor is present in an amount of 40 to 210 parts by weight;
the absorption enhancer(s) is/are present in an amount of 60 to 320 parts by weight; and
the surfactant(s) is/are present in an amount of 50 to 550 parts by weight;

preferably,

the polypeptide is present in an amount of 8 to 32 parts by weight;
the fish oil is present in an amount of 0.01 to 320 parts by weight;
the protease inhibitor is present in an amount of 70 to 200 parts by weight;
the absorption enhancer(s) is/are present in an amount of 120 to 320 parts by weight; and
the surfactant(s) is/are present in an amount of 105 to 450 parts by weight;

preferably,

the polypeptide is present in an amount of 8 to 32 parts by weight;
the fish oil is present in an amount of 50 to 320 parts by weight;
the protease inhibitor is present in an amount of 70 to 200 parts by weight;
the absorption enhancer(s) is/are present in an amount of 120 to 320 parts by weight; and
the surfactant(s) is/are present in an amount of 105 to 450 parts by weight;

preferably,

the polypeptide is present in an amount of 8 to 32 parts by weight;
the fish oil is present in an amount of 105 to 320 parts by weight;
the protease inhibitor is present in an amount of 70 to 200 parts by weight;
the absorption enhancer(s) is/are present in an amount of 120 to 320 parts by weight; and
the surfactant(s) is/are present in an amount of 105 to 320 parts by weight;

preferably,

the polypeptide is present in an amount of 8 to 32 parts by weight;
the fish oil is present in an amount of 245 to 320 parts by weight;
the protease inhibitor is present in an amount of 70 to 160 parts by weight;
the absorption enhancer(s) is/are present in an amount of 120 to 250 parts by weight; and
the surfactant(s) is/are present in an amount of 105 to 320 parts by weight.

In one embodiment, the present invention provides an oral polypeptide composition, wherein the oral polypeptide composition comprises a polypeptide, fish oil, a protease inhibitor, at least one absorption enhancer and at least one surfactant, in which

the polypeptide is present in an amount of 8 to 16 parts by weight;
the fish oil is present in an amount of 105 to 320 parts by weight;
the protease inhibitor is present in an amount of 160 to 200 parts by weight;
the absorption enhancer(s) is/are present in an amount of 120 to 320 parts by weight; and
the surfactant(s) is/are present in an amount of 245 to 320 parts by weight. or,
the polypeptide is present in an amount of 16 to 32 parts by weight;
the fish oil is present in an amount of 105 to 245 parts by weight;
the protease inhibitor is present in an amount of 70 to 200 parts by weight;
the absorption enhancer(s) is/are present in an amount of 250 to 320 parts by weight; and
the surfactant(s) is/are present in an amount of 105 to 245 parts by weight.

In one embodiment, the present invention provides an oral polypeptide composition, wherein the oral polypeptide composition comprises a polypeptide, fish oil, a protease inhibitor, at least one absorption enhancer and at least one surfactant, in which

the polypeptide is present in an amount of 1 part by weight;
the fish oil is present in an amount of 0.01 to 800 parts by weight;
the protease inhibitor is present in an amount of 0.1 to 210 parts by weight;
the absorption enhancer(s) is/are present in an amount of 0.1 to 300 parts by weight; and
the surfactant(s) is/are present in an amount of 1 to 800 parts by weight;

preferably,

the polypeptide is present in an amount of 1 part by weight;
the fish oil is present in an amount of 3 to 50 parts by weight;
the protease inhibitor is present in an amount of 1 to 30 parts by weight;
the absorption enhancer(s) is/are present in an amount of 5 to 50 parts by weight; and
the surfactant(s) is/are present in an amount of 3 to 65 parts by weight;

preferably,

the polypeptide is present in an amount of 1 part by weight;

the fish oil is present in an amount of 8 to 40 parts by weight;
the protease inhibitor is present in an amount of 3 to 20 parts by weight;
the absorption enhancer(s) is/are present in an amount of 2 to 20 parts by weight; and
the surfactant(s) is/are present in an amount of 8 to 40 parts by weight;

preferably,

the polypeptide is present in an amount of 1 part by weight;
the fish oil is present in an amount of 8 to 40 parts by weight;
the protease inhibitor is present in an amount of 3 to 10 parts by weight;
the absorption enhancer(s) is/are present in an amount of 2 to 20 parts by weight; and
the surfactant(s) is/are present in an amount of 8 to 40 parts by weight.

In one embodiment, the present invention provides an oral polypeptide composition, wherein the oral polypeptide composition comprises a polypeptide, fish oil, a protease inhibitor, at least one absorption enhancer and at least one surfactant, in which
preferably,

the polypeptide is present in an amount of 1 part by weight;
the fish oil is present in an amount of 6.3 to 40 parts by weight;
the protease inhibitor is present in an amount of 2.2 to 20 parts by weight;
the absorption enhancer(s) is/are present in an amount of 7.8 to 37.5 parts by weight; and
the surfactant(s) is/are present in an amount of 3.3 to 56.3 parts by weight;

preferably,

the polypeptide is present in an amount of 1 part by weight;
the fish oil is present in an amount of 6.6 to 40 parts by weight;
the protease inhibitor is present in an amount of 2.2 to 20 parts by weight;
the absorption enhancer(s) is/are present in an amount of 7.8 to 20 parts by weight; and
the surfactant(s) is/are present in an amount of 3.3 to 40 parts by weight;

preferably,

the polypeptide is present in an amount of 1 part by weight;
the fish oil is present in an amount of 7.7 to 40 parts by weight;
the protease inhibitor is present in an amount of 2.2 to 20 parts by weight;
the absorption enhancer(s) is/are present in an amount of 7.8 to 15 parts by weight; and
the surfactant(s) is/are present in an amount of 3.3 to 40 parts by weight;

In one embodiment, the present invention provides an oral polypeptide composition, wherein the oral polypeptide composition comprises a polypeptide, fish oil, a protease inhibitor, at least one absorption enhancer and at least one surfactant, in which
or,

the polypeptide is present in an amount of 1 part by weight;
the fish oil is present in an amount of 6.6 to 40 parts by weight;
the protease inhibitor is present in an amount of 12.5 to 20 parts by weight;
the absorption enhancer(s) is/are present in an amount of 15 to 20 parts by weight; and
the surfactant(s) is/are present in an amount of 15.3 to 40 parts by weight;
or,
the polypeptide is present in an amount of 1 part by weight;
the fish oil is present in an amount of 6.6 to 7.7 parts by weight;
the protease inhibitor is present in an amount of 2.2 to 12.5 parts by weight;
the absorption enhancer(s) is/are present in an amount of 7.8 to 20 parts by weight; and
the surfactant(s) is/are present in an amount of 3.3 to 15.3 parts by weight.

The term "mass fraction" refers to the mass fraction of a certain component in a polypeptide composition, based on the total weight of a polypeptide, fish oil, a protease inhibitor, an absorption enhancer and a surfactant in the polypeptide composition.

For example, when a polypeptide composition comprises 8 mg of recombinant human insulin, 320 mg of fish oil, 160 mg of soybean trypsin inhibitor, 120 mg of disodium ethylenediaminetetraacetate and 320 mg of Tween 80, the total weight of the polypeptide composition is 928 mg, the mass fraction of the recombinant human insulin is (8 mg/928 mg) × 100%=0.9%, and the mass fraction of the fish oil is (320 mg/928 mg) × 100%=34.5%.

In one embodiment, the present invention provides an oral polypeptide composition, wherein the oral polypeptide composition comprises a polypeptide, fish oil, a protease inhibitor, at least one absorption enhancer and at least one surfactant, in which

the polypeptide is present in a mass fraction of 0.1% to 10.0%;
the fish oil is present in a mass fraction of 0.5% to 40.0%;
the protease inhibitor is present in a mass fraction of 5.0% to 28.0%;
the absorption enhancer(s) is/are present in a mass fraction of 8.0% to 42.0%; and
the surfactant(s) is/are present in a mass fraction of 10.0% to 55.0%;
preferably,
the polypeptide is present in a mass fraction of 0.9% to 4.6%;
the fish oil is present in a mass fraction of 5.5% to 34.9%;
the protease inhibitor is present in a mass fraction of 10.0% to 22.6%;
the absorption enhancer(s) is/are present in a mass fraction of 12.9% to 36.1%; and
the surfactant(s) is/are present in a mass fraction of 15.0% to 49.6%;
preferably,
the polypeptide is present in a mass fraction of 0.9% to 4.6%;
the fish oil is present in a mass fraction of 11.9% to 34.9%;
the protease inhibitor is present in a mass fraction of 10.0% to 22.6%;
the absorption enhancer(s) is/are present in a mass fraction of 12.9% to 36.1%; and
the surfactant(s) is/are present in a mass fraction of 15.0% to 34.5%;
preferably,
the polypeptide is present in a mass fraction of 0.9% to 4.6%;
the fish oil is present in a mass fraction of 20.0% to 34.9%;
the protease inhibitor is present in a mass fraction of 10.0% to 17.2%;
the absorption enhancer(s) is/are present in a mass fraction of 12.9% to 35.6%; and
the surfactant(s) is/are present in a mass fraction of 15.0% to 34.5%;
preferably,
the polypeptide is present in a mass fraction of 0.9% to 4.6%;
the fish oil is present in a mass fraction of 34.5% to 34.9%;
the protease inhibitor is present in a mass fraction of 10.0% to 17.2%;
the absorption enhancer(s) is/are present in a mass fraction of 12.9% to 35.6%; and
the surfactant(s) is/are present in a mass fraction of 15.0% to 34.5%;
preferably,
the polypeptide is present in a mass fraction of 0.9% to 1.8%;
the fish oil is present in a mass fraction of 11.9% to 34.5%;
the protease inhibitor is present in a mass fraction of 17.2% to 22.6%;
the absorption enhancer(s) is/are present in a mass fraction of 12.9% to 36.1%; and
the surfactant(s) is/are present in a mass fraction of 27.7% to 34.5%;
preferably,
the polypeptide is present in a mass fraction of 1.8% to 4.6%;
the fish oil is present in a mass fraction of 11.9% to 34.9%;
the protease inhibitor is present in a mass fraction of 10.0% to 22.6%;
the absorption enhancer(s) is/are present in a mass fraction of 35.6% to 36.1%; and
the surfactant(s) is/are present in a mass fraction of 15.0% to 27.7%.

In one embodiment, the present invention provides an oral polypeptide composition, wherein the oral polypeptide composition comprises a polypeptide, fish oil, a protease inhibitor, at least one absorption enhancer and at least one surfactant; and in unit dosage form,

the polypeptide is present in an amount of 8 to 32 mg;
the fish oil is present in an amount of 0.01 to 320 mg;

the protease inhibitor is present in an amount of 70 to 200 mg;
the absorption enhancer(s) is/are present in an amount of 120 to 320 mg; and
the surfactant(s) is/are present in an amount of 105 to 450 mg;
preferably,
the polypeptide is present in an amount of 8 to 32 mg;
the fish oil is present in an amount of 50 to 320 mg;
the protease inhibitor is present in an amount of 70 to 200 mg;
the absorption enhancer(s) is/are present in an amount of 120 to 320 mg; and
the surfactant(s) is/are present in an amount of 105 to 450 mg;
preferably,
the polypeptide is present in an amount of 8 to 32 mg;
the fish oil is present in an amount of 105 to 320 mg;
the protease inhibitor is present in an amount of 70 to 200 mg;
the absorption enhancer(s) is/are present in an amount of 120 to 320 mg; and
the surfactant(s) is/are present in an amount of 105 to 320 mg;
preferably,
the polypeptide is present in an amount of 8 to 32 mg;
the fish oil is present in an amount of 245 to 320 mg;
the protease inhibitor is present in an amount of 70 to 160 mg;
the absorption enhancer(s) is/are present in an amount of 120 to 250 mg; and
the surfactant(s) is/are present in an amount of 105 to 320 mg.

In one embodiment, the present invention provides an oral polypeptide composition, wherein the oral polypeptide composition comprises a polypeptide, fish oil, a protease inhibitor, at least one absorption enhancer and at least one surfactant; and in unit dosage form,

the polypeptide is present in an amount of 8 to 16 mg;
the fish oil is present in an amount of 105 to 320 mg;
the protease inhibitor is present in an amount of 160 to 200 mg;
the absorption enhancer(s) is/are present in an amount of 120 to 320 mg; and
the surfactant(s) is/are present in an amount of 245 to 320 mg.
or,
the polypeptide is present in an amount of 16 to 32 mg;
the fish oil is present in an amount of 105 to 245 mg;
the protease inhibitor is present in an amount of 70 to 200 mg;
the absorption enhancer(s) is/are present in an amount of 250 to 320 mg; and
the surfactant(s) is/are present in an amount of 105 to 245 mg.

In one embodiment, the present invention provides an oral polypeptide composition, wherein the oral polypeptide composition comprises a polypeptide, fish oil, a protease inhibitor, at least one absorption enhancer and at least one surfactant; and in unit dosage form,

the polypeptide is present in an amount of 8 mg;
the fish oil is present in an amount of 320 mg;
the protease inhibitor is present in an amount of 160 mg;
the absorption enhancer(s) is/are present in an amount of 120 mg; and
the surfactant(s) is/are present in an amount of 320 mg;
or,
the polypeptide is present in an amount of 16 mg;
the fish oil is present in an amount of 105 mg;
the protease inhibitor is present in an amount of 200 mg;
the absorption enhancer(s) is/are present in an amount of 320 mg; and
the surfactant(s) is/are present in an amount of 245 mg;
or,
the polypeptide is present in an amount of 32 mg;
the fish oil is present in an amount of 245 mg;
the protease inhibitor is present in an amount of 70 mg;
the absorption enhancer(s) is/are present in an amount of 250 mg; and

the surfactant(s) is/are present in an amount of 105 mg;

or,

the polypeptide is present in an amount of 8 mg;

the fish oil is present in an amount of 50 mg;

the protease inhibitor is present in an amount of 100 mg;

the absorption enhancer(s) is/are present in an amount of 300 mg; and

the surfactant(s) is/are present in an amount of 450 mg.

In the present invention, the polypeptide, the fish oil, the protease inhibitor, the absorption enhancer and the surfactant are present in specific ratios, and the respective components act synergistically, allowing the oral polypeptide composition obtained to have high stability. Under suitable storage conditions, the oral polypeptide composition has good stability and long shelf life. The oral polypeptide composition of the present invention has high bioavailability.

In one embodiment, the present invention provides an oral polypeptide composition comprising a polypeptide, fish oil, a protease inhibitor, an absorption enhancer and a surfactant, wherein,

for example, the polypeptide is present in an amount of 0.1 to 100 parts by weight, and for example, may be present in an amount of 0.1 part by weight, 0.2 part by weight, 0.3 part by weight, 0.4 part by weight, 0.5 part by weight, 0.6 part by weight, 0.7 part by weight, 0.8 part by weight, 0.9 part by weight, 1 part by weight, 2 parts by weight, 3 parts by weight, 4 parts by weight, 5 parts by weight, 6 parts by weight, 7 parts by weight, 8 parts by weight, 9 parts by weight, 10 parts by weight, 11 parts by weight, 12 parts by weight, 13 parts by weight, 14 parts by weight, 15 parts by weight, 16 parts by weight, 17 parts by weight, 18 parts by weight, 19 parts by weight, 20 parts by weight, 21 parts by weight, 22 parts by weight, 23 parts by weight, 24 parts by weight, 25 parts by weight, 26 parts by weight, 27 parts by weight, 28 parts by weight, 29 parts by weight, 30 parts by weight, 31 parts by weight, 32 parts by weight, 33 parts by weight, 34 parts by weight, 35 parts by weight, 36 parts by weight, 37 parts by weight, 38 parts by weight, 39 parts by weight, 40 parts by weight, 41 parts by weight, 42 parts by weight, 43 parts by weight, 44 parts by weight, 45 parts by weight, 46 parts by weight, 47 parts by weight, 48 parts by weight, 49 parts by weight, 50 parts by weight, 51 parts by weight, 52 parts by weight, 53 parts by weight, 54 parts by weight, 55 parts by weight, 56 parts by weight, 57 parts by weight, 58 parts by weight, 59 parts by weight, 60 parts by weight, 61 parts by weight, 62 parts by weight, 63 parts by weight, 64 parts by weight, 65 parts by weight, 66 parts by weight, 67 parts by weight, 68 parts by weight, 69 parts by weight, 70 parts by weight, 71 parts by weight, 72 parts by weight, 73 parts by weight, 74 parts by weight, 75 parts by weight, 76 parts by weight, 77 parts by weight, 78 parts by weight, 79 parts by weight, 80 parts by weight, 81 parts by weight, 82 parts by weight, 83 parts by weight, 84 parts by weight, 85 parts by weight, 86 parts by weight, 87 parts by weight, 88 parts by weight, 89 parts by weight, 90 parts by weight, 91 parts by weight, 92 parts by weight, 93 parts by weight, 94 parts by weight, 95 parts by weight, 96 parts by weight, 97 parts by weight, 98 parts by weight, 99 parts by weight, 100 parts by weight, etc, and other specific point values within this numerical range or the values between any two points thereof can be selected and will not be described redundantly here.

The fish oil is present in an amount of 0.1 to 800 parts by weight, and for example, may be present in an amount of 0.1 part by weight, 0.2 part by weight, 0.3 part by weight, 0.4 part by weight, 0.5 part by weight, 0.6 part by weight, 0.7 part by weight, 0.8 part by weight, 0.9 part by weight, 1 part by weight, 2 parts by weight, 3 parts by weight, 4 parts by weight, 5 parts by weight, 6 parts by weight, 7 parts by weight, 8 parts by weight, 9 parts by weight, 10 parts by weight, 11 parts by weight, 12 parts by weight, 13 parts by weight, 14 parts by weight, 15 parts by weight, 16 parts by weight, 17 parts by weight, 18 parts by weight, 19 parts by weight, 20 parts by weight, 21 parts by weight, 22 parts by weight, 23 parts by weight, 24 parts by weight, 25 parts by weight, 26 parts by weight, 27 parts by weight, 28 parts by weight, 29 parts by weight, 30 parts by weight, 31 parts by weight, 32 parts by weight, 33 parts by weight, 34 parts by weight, 35 parts by weight, 36 parts by weight, 37 parts by weight, 38 parts by weight, 39 parts by weight, 40 parts by weight, 41 parts by weight, 42 parts by weight, 43 parts by weight, 44 parts by weight, 45 parts by weight, 46 parts by weight, 47 parts by weight, 48 parts by weight, 49 parts by weight, 50 parts by weight, 51 parts by weight, 52 parts by weight, 53 parts by weight, 54 parts by weight, 55 parts by weight, 56 parts by weight, 57 parts by weight, 58 parts by weight, 59 parts by weight, 60 parts by weight, 61 parts by weight, 62 parts by weight, 63 parts by weight, 64 parts by weight, 65 parts by weight, 66 parts by weight, 67 parts by weight, 68 parts by weight, 69 parts by weight, 70 parts by weight, 71 parts by weight, 72 parts by weight, 73 parts by weight, 74 parts by weight, 75 parts by weight, 76 parts by weight, 77 parts by weight, 78 parts by weight, 79 parts by weight, 80 parts by weight, 81 parts by weight, 82 parts by weight, 83 parts by weight, 84 parts by weight, 85 parts by weight, 86 parts by weight, 87 parts by weight, 88 parts by weight, 89 parts by weight, 90 parts by weight, 91 parts by weight, 92 parts by weight, 93 parts by weight, 94 parts by weight, 95 parts by weight, 96 parts by weight, 97 parts by weight, 98 parts by weight, 99 parts by weight, 100 parts by weight, 101 parts by weight, 102 parts by weight, 103 parts by weight, 104 parts by weight, 105 parts by weight, 106 parts by weight, 107 parts by weight, 108 parts by weight, 109 parts by weight, 110 parts by weight, 111 parts by weight, 112 parts by weight, 113 parts by weight, 114 parts by weight, 115 parts by weight, 116 parts by weight, 117 parts by weight, 118 parts by weight, 119 parts by weight, 120 parts by weight, 121 parts by weight, 122 parts by weight, 123 parts by weight, 124 parts by weight, 125 parts by weight, 126 parts by weight, 127 parts by weight, 128 parts by weight, 129 parts by weight, 130 parts by weight, 131 parts by weight, 132 parts by weight, 133 parts by weight, 134 parts by weight, 135 parts by weight, 136 parts by weight, 137 parts by weight, 138 parts by weight, 139 parts by weight, 140 parts by weight, 141 parts by weight, 142 parts by weight, 143 parts by weight, 144 parts by weight, 145 parts by weight, 146 parts by weight, 147 parts by weight, 148 parts by weight, 149 parts by weight, 150 parts by weight, 151 parts by weight, 152 parts by weight, 153 parts by weight, 154 parts by weight, 155 parts by weight, 156 parts by weight, 157 parts by weight, 158 parts by weight, 159 parts by weight, 160 parts by weight, 161 parts by weight, 162 parts by weight, 163 parts by weight, 164 parts by weight, 165 parts by weight, 166 parts by weight, 167 parts by weight, 168 parts by weight, 169 parts by weight, 170 parts by weight, 171 parts by weight, 172 parts by weight, 173 parts by weight, 174 parts by weight, 175 parts by weight, 176 parts by weight, 177 parts by weight, 178 parts by weight, 179 parts by weight, 180 parts by weight, 181 parts by weight, 182 parts by weight, 183 parts by weight, 184 parts by weight, 185 parts by weight, 186 parts by weight, 187 parts by weight, 188 parts by weight, 189 parts by weight, 190 parts by weight, 191 parts by weight, 192 parts by weight, 193 parts by weight, 194 parts by weight, 195 parts by weight, 196 parts by weight, 197 parts by weight, 198 parts by weight, 199 parts by weight, 200 parts by weight, 201 parts by weight, 202 parts by weight, 203 parts by weight, 204 parts by weight, 205 parts by weight, 206 parts by weight, 207 parts by weight, 208 parts by weight, 209 parts by weight, 210 parts by weight, 211 parts by weight, 212 parts by weight, 213 parts by weight, 214 parts by weight, 215 parts by weight, 216 parts by weight, 217 parts by weight, 218 parts by weight, 219 parts by weight, 220 parts by weight, 221 parts by weight, 222 parts by weight, 223 parts by weight, 224 parts by weight, 225 parts by weight, 226 parts by weight, 227 parts by weight, 228 parts by weight, 229 parts by weight, 230 parts by weight, 231 parts by weight, 232 parts by weight, 233 parts by weight, 234 parts by weight, 235 parts by weight, 236 parts by weight, 237 parts by weight, 238 parts by weight, 239 parts by weight, 240 parts by weight, 241 parts by weight, 242 parts by weight, 243 parts by weight, 244 parts by weight, 245 parts by weight, 246 parts by weight, 247 parts by weight, 248 parts by weight, 249 parts by weight, 250 parts by weight, 251 parts by weight, 252 parts by weight, 253 parts by weight, 254 parts by weight, 255 parts by weight, 256 parts by weight, 257 parts by weight, 258 parts by weight, 259 parts by weight, 260 parts by weight, 261 parts by weight, 262 parts by weight, 263 parts by weight, 264 parts by weight, 265 parts by weight, 266 parts by weight, 267 parts by weight, 268 parts by weight, 269 parts by weight, 270 parts by weight, 271 parts by weight, 272 parts by weight, 273 parts by weight, 274 parts by weight, 275 parts by weight, 276 parts by weight, 277 parts by weight, 278 parts by weight, 279 parts by weight, 280 parts by weight, 281 parts by weight, 282 parts by weight, 283 parts by weight, 284 parts by weight, 285 parts by weight, 286 parts by weight, 287 parts by weight, 288 parts by weight, 289 parts by weight, 290 parts by weight, 291 parts by weight, 292 parts by weight, 293 parts by weight, 294 parts by weight, 295 parts by weight, 296 parts by weight, 297 parts by weight, 298 parts by weight, 299 parts by weight, 300 parts by weight, 301 parts by weight, 302 parts by weight, 303 parts by weight, 304 parts by weight, 305 parts by weight, 306 parts by weight, 307 parts by weight, 308 parts by weight, 309 parts by weight, 310 parts by weight, 311 parts by weight, 312 parts by weight, 313 parts by weight, 314 parts by weight, 315 parts by weight, 316 parts by weight, 317 parts by weight, 318 parts by weight, 319 parts by weight, 320 parts by weight, 321 parts by weight, 322 parts by weight, 323 parts by weight, 324 parts by weight, 325 parts by weight, 326 parts by weight, 327 parts by weight, 328 parts by weight, 329 parts by weight, 330 parts by weight, 331 parts by weight, 332 parts by weight, 333 parts by weight, 334 parts by weight, 335 parts by weight, 336 parts by weight, 337 parts by weight, 338 parts by weight, 339 parts by weight, 340 parts by weight, 341 parts by weight, 342 parts by weight, 343 parts by weight, 344 parts by weight, 345 parts by weight, 346 parts by weight, 347 parts by weight, 348 parts by weight, 349 parts by weight, 350 parts by weight, 351 parts by weight, 352 parts by weight, 353 parts by weight, 354 parts by weight, 355 parts by weight, 356 parts by weight, 357 parts by weight, 358 parts by weight, 359 parts by weight, 360 parts by weight, 361 parts by weight, 362 parts by weight, 363 parts by weight, 364 parts by weight, 365 parts by weight, 366 parts by weight, 367 parts by weight, 368 parts by weight, 369 parts by weight, 370 parts by weight, 371 parts by weight, 372 parts by weight, 373 parts by weight, 374 parts by weight, 375 parts by weight, 376 parts by weight, 377 parts by weight, 378 parts by weight, 379 parts by weight, 380 parts by weight, 381 parts by weight, 382 parts by weight, 383 parts by weight, 384 parts by weight, 385 parts by weight, 386 parts by weight, 387 parts by weight, 388 parts by weight, 389 parts by weight, 390 parts by weight, 391 parts by weight, 392 parts by weight, 393 parts by weight, 394 parts by weight, 395 parts by weight, 396 parts by weight, 397 parts by weight, 398 parts by weight, 399 parts by weight, 400 parts by weight, 401 parts by weight, 402 parts by weight, 403 parts by weight, 404 parts by weight, 405 parts by weight, 406 parts by weight, 407 parts by weight, 408 parts by weight, 409 parts by weight, 410 parts by weight, 411 parts by weight, 412 parts by weight, 413 parts by weight, 414 parts by weight, 415 parts by weight, 416 parts by weight, 417 parts by weight, 418 parts by weight, 419 parts by weight, 420 parts by weight, 421 parts by weight, 422 parts by weight, 423 parts by weight, 424 parts by weight, 425 parts by weight, 426 parts by weight, 427 parts by weight, 428 parts by weight, 429 parts by weight, 430 parts by weight, 431 parts by weight, 432 parts by weight, 433 parts by weight, 434 parts by weight, 435 parts by weight, 436 parts by weight, 437 parts by weight, 438 parts by weight, 439 parts by weight, 440 parts by weight, 441 parts by weight, 442 parts by weight, 443 parts by weight, 444 parts by weight, 445 parts by weight, 446 parts by weight, 447 parts by weight, 448 parts by weight, 449 parts by weight, 450 parts by weight, 451 parts by weight, 452 parts by weight, 453 parts by weight, 454 parts by weight, 455 parts by weight, 456 parts by weight, 457 parts by weight, 458 parts by weight, 459 parts by weight, 460 parts by weight, 461 parts by weight, 462 parts by weight, 463 parts by weight, 464 parts by weight, 465 parts by weight, 466 parts by weight, 467 parts by weight, 468 parts by weight, 469 parts by weight, 470 parts by weight, 471 parts by weight, 472 parts by weight, 473 parts by weight, 474 parts by weight, 475 parts by weight, 476 parts by weight, 477 parts by weight, 478 parts by weight, 479 parts by weight, 480 parts by weight, 481 parts by weight, 482 parts by weight, 483 parts by weight, 484 parts by weight, 485 parts by weight, 486 parts by weight, 487 parts by weight, 488 parts by weight, 489 parts by weight, 490 parts by weight, 491 parts by weight, 492 parts by weight, 493 parts by weight, 494 parts by weight, 495 parts by weight, 496 parts by weight, 497 parts by weight, 498 parts by weight, 499 parts by weight, 500 parts by weight, 501 parts by weight, 502 parts by weight, 503 parts by weight, 504 parts by weight, 505 parts by weight, 506 parts by weight, 507 parts by weight, 508 parts by weight, 509 parts by weight, 510 parts by weight, 511 parts by weight, 512 parts by weight, 513 parts by weight, 514 parts by weight, 515 parts by weight, 516 parts by weight, 517 parts by weight, 518 parts by weight, 519 parts by weight, 520 parts by weight, 521 parts by weight, 522 parts by weight, 523 parts by weight, 524 parts by weight, 525 parts by weight, 526 parts by weight, 527 parts by weight, 528 parts by weight, 529 parts by weight, 530 parts by weight, 531 parts by weight, 532 parts by weight, 533 parts by weight, 534 parts by weight, 535 parts by weight, 536 parts by weight, 537 parts by weight, 538 parts by weight, 539 parts by weight, 540 parts by weight, 541 parts by weight, 542 parts by weight, 543 parts by weight, 544 parts by weight, 545 parts by weight, 546 parts by weight, 547 parts by weight, 548 parts by weight, 549 parts by weight, 550 parts by weight, 551 parts by weight, 552 parts by weight, 553 parts by weight, 554 parts by weight, 555 parts by weight, 556 parts by weight, 557 parts by weight, 558 parts by weight, 559 parts by weight, 560 parts by weight, 561 parts by weight, 562 parts by weight, 563 parts by weight, 564 parts by weight, 565 parts by weight, 566 parts by weight, 567 parts by weight, 568 parts by weight, 569 parts by weight, 570 parts by weight, 571 parts by weight, 572 parts by weight, 573 parts by weight, 574 parts by weight, 575 parts by weight, 576 parts by weight, 577 parts by weight, 578 parts by weight, 579 parts by weight, 580 parts by weight, 581 parts by weight, 582 parts by weight, 583 parts by weight, 584 parts by weight, 585 parts by weight, 586 parts by weight, 587 parts by weight, 588 parts by weight, 589 parts by weight, 590 parts by weight, 591 parts by weight, 592 parts by weight, 593 parts by weight, 594 parts by weight, 595 parts by weight, 596 parts by weight, 597 parts by weight, 598 parts by weight, 599 parts by weight, 600 parts by weight, 601 parts by weight, 602 parts by weight, 603 parts by weight, 604 parts by weight, 605 parts by weight, 606 parts by weight, 607 parts by weight, 608 parts by weight, 609 parts by weight, 610 parts by weight, 611 parts by weight, 612 parts by weight, 613 parts by weight, 614 parts by weight, 615 parts by weight, 616 parts by weight, 617 parts by weight, 618 parts by weight, 619 parts by weight, 620 parts by weight, 621 parts by weight, 622 parts by weight, 623 parts by weight, 624 parts by weight, 625 parts by weight, 626 parts by weight, 627 parts by weight, 628 parts by weight, 629 parts by weight, 630 parts by weight, 631 parts by weight, 632 parts by weight, 633 parts by weight, 634 parts by weight, 635 parts by weight, 636 parts by weight, 637 parts by weight, 638 parts by weight, 639 parts by weight, 640 parts by weight, 641 parts by weight, 642 parts by weight, 643 parts by weight, 644 parts by weight, 645 parts by weight, 646 parts by weight, 647 parts by weight, 648 parts by weight, 649 parts by weight, 650 parts by weight, 651 parts by weight, 652 parts by weight, 653 parts by weight, 654 parts by weight, 655 parts by weight, 656 parts by weight, 657 parts by weight, 658 parts by weight, 659 parts by weight, 660 parts by weight, 661 parts by weight, 662 parts by weight, 663 parts by weight, 664 parts by weight, 665 parts by weight, 666 parts by weight, 667 parts by weight, 668 parts by weight, 669 parts by weight, 670 parts by weight, 671 parts by weight, 672 parts by weight, 673 parts by weight, 674 parts by weight, 675 parts by weight, 676 parts by weight, 677 parts by weight, 678 parts by weight, 679 parts by weight, 680 parts by weight, 681 parts by weight, 682 parts by weight, 683 parts by weight, 684 parts by weight, 685 parts by weight, 686 parts by weight, 687 parts by weight, 688 parts by weight, 689 parts by weight, 690 parts by weight, 691 parts by weight, 692 parts by weight, 693 parts by weight, 694 parts by weight, 695 parts by weight, 696 parts by weight, 697 parts by weight, 698 parts by weight, 699 parts by weight, 700 parts by weight, 701 parts by weight, 702 parts by weight, 703 parts by weight, 704 parts by weight, 705 parts by weight, 706 parts by weight, 707 parts by weight, 708 parts by weight, 709 parts by weight, 710 parts by weight, 711 parts by weight, 712 parts by weight, 713 parts by weight, 714 parts by weight, 715 parts by weight, 716 parts by weight, 717 parts by weight, 718 parts by weight, 719 parts by weight, 720 parts by weight, 721 parts by weight, 722 parts by weight, 723 parts by weight, 724 parts by weight, 725 parts by weight, 726 parts by weight, 727 parts by weight, 728 parts by weight, 729 parts by weight, 730 parts by weight, 731 parts by weight, 732 parts by weight, 733 parts by weight, 734 parts by weight, 735 parts by weight, 736 parts by weight, 737 parts by weight, 738 parts by weight, 739 parts by weight, 740 parts by weight, 741 parts by weight, 742 parts by weight, 743 parts by weight, 744 parts by weight, 745 parts by weight, 746 parts by weight, 747 parts by weight, 748 parts by weight, 749 parts by weight, 750 parts by weight, 751 parts by weight, 752 parts by weight, 753 parts by weight, 754 parts by weight, 755 parts by weight, 756 parts by weight, 757 parts by weight, 758 parts by weight, 759 parts by weight, 760 parts by weight, 761 parts by weight, 762 parts by weight, 763 parts by weight, 764 parts by weight, 765 parts by weight, 766 parts by weight, 767 parts by weight, 768 parts by weight, 769 parts by weight, 770 parts by weight, 771 parts by weight, 772 parts by weight, 773 parts by weight, 774 parts by weight, 775 parts by weight, 776 parts by weight, 777 parts by weight, 778 parts by weight, 779 parts by weight, 780 parts by weight, 781 parts by weight, 782 parts by weight, 783 parts by weight, 784 parts by weight, 785 parts by weight, 786 parts by weight, 787 parts by weight, 788 parts by weight, 789 parts by weight, 790 parts by weight, 791 parts by weight, 792 parts by weight, 793 parts by weight, 794 parts by weight, 795 parts by weight, 796 parts by weight, 797 parts by weight, 798 parts by weight, 799 parts by weight, 800 parts by weight, etc, and other specific point values within this numerical range or the values between any two points thereof can be selected and will not be described redundantly here.

The protease inhibitor is present in an amount of 1 to 400 parts by weight, and for example, may be present in an amount of 1 part by weight, 2 parts by weight, 3 parts by weight, 4 parts by weight, 5 parts by weight, 6 parts by weight, 7 parts by weight, 8 parts by weight, 9 parts by weight, 10 parts by weight, 11 parts by weight, 12 parts by weight, 13 parts by weight, 14 parts by weight, 15 parts by weight, 16 parts by weight, 17 parts by weight, 18 parts by weight, 19 parts by weight, 20 parts by weight, 21 parts by weight, 22 parts by weight, 23 parts by weight, 24 parts by weight, 25 parts by weight, 26 parts by weight, 27 parts by weight, 28 parts by weight, 29 parts by weight, 30 parts by weight, 31 parts by weight, 32 parts by weight, 33 parts by weight, 34 parts by weight, 35 parts by weight, 36 parts by weight, 37 parts by weight, 38 parts by weight, 39 parts by weight, 40 parts by weight, 41 parts by weight, 42 parts by weight, 43 parts by weight, 44 parts by weight, 45 parts by weight, 46 parts by weight, 47 parts by weight, 48 parts by weight, 49 parts by weight, 50 parts by weight, 51 parts by weight, 52 parts by weight, 53 parts by weight, 54 parts by weight, 55 parts by weight, 56 parts by weight, 57 parts by weight, 58 parts by weight, 59 parts by weight, 60 parts by weight, 61 parts by weight, 62 parts by weight, 63 parts by weight, 64 parts by weight, 65 parts by weight, 66 parts by weight, 67 parts by weight, 68 parts by weight, 69 parts by weight, 70 parts by weight, 71 parts by weight, 72 parts by weight, 73 parts by weight, 74 parts by weight, 75 parts by weight, 76 parts by weight, 77 parts by weight, 78 parts by weight, 79 parts by weight, 80 parts by weight, 81 parts by weight, 82 parts by weight, 83 parts by weight, 84 parts by weight, 85 parts by weight, 86 parts by weight, 87 parts by weight, 88 parts by weight, 89 parts by weight, 90 parts by weight, 91 parts by weight, 92 parts by weight, 93 parts by weight, 94 parts by weight, 95 parts by weight, 96 parts by weight, 97 parts by weight, 98 parts by weight, 99 parts by weight, 100 parts by weight, 101 parts by weight, 102 parts by weight, 103 parts by weight, 104 parts by weight, 105 parts by weight, 106 parts by weight, 107 parts by weight, 108 parts by weight, 109 parts by weight, 110 parts by weight, 111 parts by weight, 112 parts by weight, 113 parts by weight, 114 parts by weight, 115 parts by weight, 116 parts by weight, 117 parts by weight, 118 parts by weight, 119 parts by weight, 120 parts by weight, 121 parts by weight, 122 parts by weight, 123 parts by weight, 124 parts by weight, 125 parts by weight, 126 parts by weight, 127 parts by weight, 128 parts by weight, 129 parts by weight, 130 parts by weight, 131 parts by weight, 132 parts by weight, 133 parts by weight, 134 parts by weight, 135 parts by weight, 136 parts by weight, 137 parts by weight, 138 parts by weight, 139 parts by weight, 140 parts by weight, 141 parts by weight, 142 parts by weight, 143 parts by weight, 144 parts by weight, 145 parts by weight, 146 parts by weight, 147 parts by weight, 148 parts by weight, 149 parts by weight, 150 parts by weight, 151 parts by weight, 152 parts by weight, 153 parts by weight, 154 parts by weight, 155 parts by weight, 156 parts by weight, 157 parts by weight, 158 parts by weight, 159 parts by weight, 160 parts by weight, 161 parts by weight, 162 parts by weight, 163 parts by weight, 164 parts by weight, 165 parts by weight, 166 parts by weight, 167 parts by weight, 168 parts by weight, 169 parts by weight, 170 parts by weight, 171 parts by weight, 172 parts by weight, 173 parts by weight, 174 parts by weight, 175 parts by weight, 176 parts by weight, 177 parts by weight, 178 parts by weight, 179 parts by weight, 180 parts by weight, 181 parts by weight, 182 parts by weight, 183 parts by weight, 184 parts by weight, 185 parts by weight, 186 parts by weight, 187 parts by weight, 188 parts by weight, 189 parts by weight, 190 parts by weight, 191 parts by weight, 192 parts by weight, 193 parts by weight, 194 parts by weight, 195 parts by weight, 196 parts by weight, 197 parts by weight, 198 parts by weight, 199 parts by weight, 200 parts by weight, 201 parts by weight, 202 parts by weight, 203 parts by weight, 204 parts by weight, 205 parts by weight, 206 parts by weight, 207 parts by weight, 208 parts by weight, 209 parts by weight, 210 parts by weight, 211 parts by weight, 212 parts by weight, 213 parts by weight, 214 parts by weight, 215 parts by weight, 216 parts by weight, 217 parts by weight, 218 parts by weight, 219 parts by weight, 220 parts by weight, 221 parts by weight, 222 parts by weight, 223 parts by weight, 224 parts by weight, 225 parts by weight, 226 parts by weight, 227 parts by weight, 228 parts by weight, 229 parts by weight, 230 parts by weight, 231 parts by weight, 232 parts by weight, 233 parts by weight, 234 parts by weight, 235 parts by weight, 236 parts by weight, 237 parts by weight, 238 parts by weight, 239 parts by weight, 240 parts by weight, 241 parts by weight, 242 parts by weight, 243 parts by weight, 244 parts by weight, 245 parts by weight, 246 parts by weight, 247 parts by weight, 248 parts by weight, 249 parts by weight, 250 parts by weight, 251 parts by weight, 252 parts by weight, 253 parts by weight, 254 parts by weight, 255 parts by weight, 256 parts by weight, 257 parts by weight, 258 parts by weight, 259 parts by weight, 260 parts by weight, 261 parts by weight, 262 parts by weight, 263 parts by weight, 264 parts by weight, 265 parts by weight, 266 parts by weight, 267 parts by weight, 268 parts by weight, 269 parts by weight, 270 parts by weight, 271 parts by weight, 272 parts by weight, 273 parts by weight, 274 parts by weight, 275 parts by weight, 276 parts by weight, 277 parts by weight, 278 parts by weight, 279 parts by weight, 280 parts by weight, 281 parts by weight, 282 parts by weight, 283 parts by weight, 284 parts by weight, 285 parts by weight, 286 parts by weight, 287 parts by weight, 288 parts by weight, 289 parts by weight, 290 parts by weight, 291 parts by weight, 292 parts by weight, 293 parts by weight, 294 parts by weight, 295 parts by weight, 296 parts by weight, 297 parts by weight, 298 parts by weight, 299 parts by weight, 300 parts by weight, 301 parts by weight, 302 parts by weight, 303 parts by weight, 304 parts by weight, 305 parts by weight, 306 parts by weight, 307 parts by weight, 308 parts by weight, 309 parts by weight, 310 parts by weight, 311 parts by weight, 312 parts by weight, 313 parts by weight, 314 parts by weight, 315 parts by weight, 316 parts by weight, 317 parts by weight, 318 parts by weight, 319 parts by weight, 320 parts by weight, 321 parts by weight, 322 parts by weight, 323 parts by weight, 324 parts by weight, 325 parts by weight, 326 parts by weight, 327 parts by weight, 328 parts by weight, 329 parts by weight, 330 parts by weight, 331 parts by weight, 332 parts by weight, 333 parts by weight, 334 parts by weight, 335 parts by weight, 336 parts by weight, 337 parts by weight, 338 parts by weight, 339 parts by weight, 340 parts by weight, 341 parts by weight, 342 parts by weight, 343 parts by weight, 344 parts by weight, 345 parts by weight, 346 parts by weight, 347 parts by weight, 348 parts by weight, 349 parts by weight, 350 parts by weight, 351 parts by weight, 352 parts by weight, 353 parts by weight, 354 parts by weight, 355 parts by weight, 356 parts by weight, 357 parts by weight, 358 parts by weight, 359 parts by weight, 360 parts by weight, 361 parts by weight, 362 parts by weight, 363 parts by weight, 364 parts by weight, 365 parts by weight, 366 parts by weight, 367 parts by weight, 368 parts by weight, 369 parts by weight, 370 parts by weight, 371 parts by weight, 372 parts by weight, 373 parts by weight, 374 parts by weight, 375 parts by weight, 376 parts by weight, 377 parts by weight, 378 parts by weight, 379 parts by weight, 380 parts by weight, 381 parts by weight, 382 parts by weight, 383 parts by weight, 384 parts by weight, 385 parts by weight, 386 parts by weight, 387 parts by weight, 388 parts by weight, 389 parts by weight, 390 parts by weight, 391 parts by weight, 392 parts by weight, 393 parts by weight, 394 parts by weight, 395 parts by weight, 396 parts by weight, 397 parts by weight, 398 parts by weight, 399 parts by weight, 400 parts by weight, etc, and other specific point values within this numerical range or the values between any two points thereof can be selected and will not be described redundantly here.

The absorption enhancer is present in an amount of 0.1 to 300 parts by weight, and for example, may be present in an amount of 1 part by weight, 2 parts by weight, 3 parts by weight, 4 parts by weight, 5 parts by weight, 6 parts by weight, 7 parts by weight, 8 parts by weight, 9 parts by weight, 10 parts by weight, 11 parts by weight, 12 parts by weight, 13 parts by weight, 14 parts by weight, 15 parts by weight, 16 parts by weight, 17 parts by weight, 18 parts by weight, 19 parts by weight, 20 parts by weight, 21 parts by weight, 22 parts by weight, 23 parts by weight, 24 parts by weight, 25 parts by weight, 26 parts by weight, 27 parts by weight, 28 parts by weight, 29 parts by weight, 30 parts by weight, 31 parts by weight, 32 parts by weight, 33 parts by weight, 34 parts by weight, 35 parts by weight, 36 parts by weight, 37 parts by weight, 38 parts by weight, 39 parts by weight, 40 parts by weight, 41 parts by weight, 42 parts by weight, 43 parts by weight, 44 parts by weight, 45 parts by weight, 46 parts by weight, 47 parts by weight, 48 parts by weight, 49 parts by weight, 50 parts by weight, 51 parts by weight, 52 parts by weight, 53 parts by weight, 54 parts by weight, 55 parts by weight, 56 parts by weight, 57 parts by weight, 58 parts by weight, 59 parts by weight, 60 parts by weight, 61 parts by weight, 62 parts by weight, 63 parts by weight, 64 parts by weight, 65 parts by weight, 66 parts by weight, 67 parts by weight, 68 parts by weight, 69 parts by weight, 70 parts by weight, 71 parts by weight, 72 parts by weight, 73 parts by weight, 74 parts by weight, 75 parts by weight, 76 parts by weight, 77 parts by weight, 78 parts by weight, 79 parts by weight, 80 parts by weight, 81 parts by weight, 82 parts by weight, 83 parts by weight, 84 parts by weight, 85 parts by weight, 86 parts by weight, 87 parts by weight, 88 parts by weight, 89 parts by weight, 90 parts by weight, 91 parts by weight, 92 parts by weight, 93 parts by weight, 94 parts by weight, 95 parts by weight, 96 parts by weight, 97 parts by weight, 98 parts by weight, 99 parts by weight, 100 parts by weight, 101 parts by weight, 102 parts by weight, 103 parts by weight, 104 parts by weight, 105 parts by weight, 106 parts by weight, 107 parts by weight, 108 parts by weight, 109 parts by weight, 110 parts by weight, 111 parts by weight, 112 parts by weight, 113 parts by weight, 114 parts by weight, 115 parts by weight, 116 parts by weight, 117 parts by weight, 118 parts by weight, 119 parts by weight, 120 parts by weight, 121 parts by weight, 122 parts by weight, 123 parts by weight, 124 parts by weight, 125 parts by weight, 126 parts by weight, 127 parts by weight, 128 parts by weight, 129 parts by weight, 130 parts by weight, 131 parts by weight, 132 parts by weight, 133 parts by weight, 134 parts by weight, 135 parts by weight, 136 parts by weight, 137 parts by weight, 138 parts by weight, 139 parts by weight, 140 parts by weight, 141 parts by weight, 142 parts by weight, 143 parts by weight, 144 parts by weight, 145 parts by weight, 146 parts by weight, 147 parts by weight, 148 parts by weight, 149 parts by weight, 150 parts by weight, 151 parts by weight, 152 parts by weight, 153 parts by weight, 154 parts by weight, 155 parts by weight, 156 parts by weight, 157 parts by weight, 158 parts by weight, 159 parts by weight, 160 parts by weight, 161 parts by weight, 162 parts by weight, 163 parts by weight, 164 parts by weight, 165 parts by weight, 166 parts by weight, 167 parts by weight, 168 parts by weight, 169 parts by weight, 170 parts by weight, 171 parts by weight, 172 parts by weight, 173 parts by weight, 174 parts by weight, 175 parts by weight, 176 parts by weight, 177 parts by weight, 178 parts by weight, 179 parts by weight, 180 parts by weight, 181 parts by weight, 182 parts by weight, 183 parts by weight, 184 parts by weight, 185 parts by weight, 186 parts by weight, 187 parts by weight, 188 parts by weight, 189 parts by weight, 190 parts by weight, 191 parts by weight, 192 parts by weight, 193 parts by weight, 194 parts by weight, 195 parts by weight, 196 parts by weight, 197 parts by weight, 198 parts by weight, 199 parts by weight, 200 parts by weight, 201 parts by weight, 202 parts by weight, 203 parts by weight, 204 parts by weight, 205 parts by weight, 206 parts by weight, 207 parts by weight, 208 parts by weight, 209 parts by weight, 210 parts by weight, 211 parts by weight, 212 parts by weight, 213 parts by weight, 214 parts by weight, 215 parts by weight, 216 parts by weight, 217 parts by weight, 218 parts by weight, 219 parts by weight, 220 parts by weight, 221 parts by weight, 222 parts by weight, 223 parts by weight, 224 parts by weight, 225 parts by weight, 226 parts by weight, 227 parts by weight, 228 parts by weight, 229 parts by weight, 230 parts by weight, 231 parts by weight, 232 parts by weight, 233 parts by weight, 234 parts by weight, 235 parts by weight, 236 parts by weight, 237 parts by weight, 238 parts by weight, 239 parts by weight, 240 parts by weight, 241 parts by weight, 242 parts by weight, 243 parts by weight, 244 parts by weight, 245 parts by weight, 246 parts by weight, 247 parts by weight, 248 parts by weight, 249 parts by weight, 250 parts by weight, 251 parts by weight, 252 parts by weight, 253 parts by weight, 254 parts by weight, 255 parts by weight, 256 parts by weight, 257 parts by weight, 258 parts by weight, 259 parts by weight, 260 parts by weight, 261 parts by weight, 262 parts by weight, 263 parts by weight, 264 parts by weight, 265 parts by weight, 266 parts by weight, 267 parts by weight, 268 parts by weight, 269 parts by weight, 270 parts by weight, 271 parts by weight, 272 parts by weight, 273 parts by weight, 274 parts by weight, 275 parts by weight, 276 parts by weight, 277 parts by weight, 278 parts by weight, 279 parts by weight, 280 parts by weight, 281 parts by weight, 282 parts by weight, 283 parts by weight, 284 parts by weight, 285 parts by weight, 286 parts by weight, 287 parts by weight, 288 parts by weight, 289 parts by weight, 290 parts by weight, 291 parts by weight, 292 parts by weight, 293 parts by weight, 294 parts by weight, 295 parts by weight, 296 parts by weight, 297 parts by weight, 298 parts by weight, 299 parts by weight, 300 parts by weight, 301 parts by weight, 302 parts by weight, 303 parts by weight, 304 parts by weight, 305 parts by weight, 306 parts by weight, 307 parts by weight, 308 parts by weight, 309 parts by weight, 310 parts by weight, 311 parts by weight, 312 parts by weight, 313 parts by weight, 314 parts by weight, 315 parts by weight, 316 parts by weight, 317 parts by weight, 318 parts by weight, 319 parts by weight, 320 parts by weight, 321 parts by weight, 322 parts by weight, 323 parts by weight, 324 parts by weight, 325 parts by weight, 326 parts by weight, 327 parts by weight, 328 parts by weight, 329 parts by weight, 330 parts by weight, 331 parts by weight, 332 parts by weight, 333 parts by weight, 334 parts by weight, 335 parts by weight, 336 parts by weight, 337 parts by weight, 338 parts by weight, 339 parts by weight, 340 parts by weight, 341 parts by weight, 342 parts by weight, 343 parts by weight, 344 parts by weight, 345 parts by weight, 346 parts by weight, 347 parts by weight, 348 parts by weight, 349 parts by weight, 350 parts by weight, 351 parts by weight, 352 parts by weight, 353 parts by weight, 354 parts by weight, 355 parts by weight, 356 parts by weight, 357 parts by weight, 358 parts by weight, 359 parts by weight, 360 parts by weight, 361 parts by weight, 362 parts by weight, 363 parts by weight, 364 parts by weight, 365 parts by weight, 366 parts by weight, 367 parts by weight, 368 parts by weight, 369 parts by weight, 370 parts by weight, 371 parts by weight, 372 parts by weight, 373 parts by weight, 374 parts by weight, 375 parts by weight, 376 parts by weight, 377 parts by weight, 378 parts by weight, 379 parts by weight, 380 parts by weight, 381 parts by weight, 382 parts by weight, 383 parts by weight, 384 parts by weight, 385 parts by weight, 386 parts by weight, 387 parts by weight, 388 parts by weight, 389 parts by weight, 390 parts by weight, 391 parts by weight, 392 parts by weight, 393 parts by weight, 394 parts by weight, 395 parts by weight, 396 parts by weight, 397 parts by weight, 398 parts by weight, 399 parts by weight, 400 parts by weight, 401 parts by weight, 402 parts by weight, 403 parts by weight, 404 parts by weight, 405 parts by weight, 406 parts by weight, 407 parts by weight, 408 parts by weight, 409 parts by weight, 410 parts by weight, 411 parts by weight, 412 parts by weight, 413 parts by weight, 414 parts by weight, 415 parts by weight, 416 parts by weight, 417 parts by weight, 418 parts by weight, 419 parts by weight, 420 parts by weight, 421 parts by weight, 422 parts by weight, 423 parts by weight, 424 parts by weight, 425 parts by weight, 426 parts by weight, 427 parts by weight, 428 parts by weight, 429 parts by weight, 430 parts by weight, 431 parts by weight, 432 parts by weight, 433 parts by weight, 434 parts by weight, 435 parts by weight, 436 parts by weight, 437 parts by weight, 438 parts by weight, 439 parts by weight, 440 parts by weight, 441 parts by weight, 442 parts by weight, 443 parts by weight, 444 parts by weight, 445 parts by weight, 446 parts by weight, 447 parts by weight, 448 parts by weight, 449 parts by weight, 450 parts by weight, 451 parts by weight, 452 parts by weight, 453 parts by weight, 454 parts by weight, 455 parts by weight, 456 parts by weight, 457 parts by weight, 458 parts by weight, 459 parts by weight, 460 parts by weight, 461 parts by weight, 462 parts by weight, 463 parts by weight, 464 parts by weight, 465 parts by weight, 466 parts by weight, 467 parts by weight, 468 parts by weight, 469 parts by weight, 470 parts by weight, 471 parts by weight, 472 parts by weight, 473 parts by weight, 474 parts by weight, 475 parts by weight, 476 parts by weight, 477 parts by weight, 478 parts by weight, 479 parts by weight, 480 parts by weight, 481 parts by weight, 482 parts by weight, 483 parts by weight, 484 parts by weight, 485 parts by weight, 486 parts by weight, 487 parts by weight, 488 parts by weight, 489 parts by weight, 490 parts by weight, 491 parts by weight, 492 parts by weight, 493 parts by weight, 494 parts by weight, 495 parts by weight, 496 parts by weight, 497 parts by weight, 498 parts by weight, 499 parts by weight, 500 parts by weight, etc, and other specific point values within this numerical range or the values between any two points thereof can be selected and will not be described redundantly here.

The surfactant is present in an amount of 0.1 to 800 parts by weight, and for example, may be present in an amount of 0.1 part by weight, 0.2 part by weight, 0.3 part by weight, 0.4 part by weight, 0.5 part by weight, 0.6 part by weight, 0.7 part by weight, 0.8 part by weight, 0.9 part by weight, 1 part by weight, 2 parts by weight, 3 parts by weight, 4 parts by weight, 5 parts by weight, 6 parts by weight, 7 parts by weight, 8 parts by weight, 9 parts by weight, 10 parts by weight, 11 parts by weight, 12 parts by weight, 13 parts by weight, 14 parts by weight, 15 parts by weight, 16 parts by weight, 17 parts by weight, 18 parts by weight, 19 parts by weight, 20 parts by weight, 21 parts by weight, 22 parts by weight, 23 parts by weight, 24 parts by weight, 25 parts by weight, 26 parts by weight, 27 parts by weight, 28 parts by weight, 29 parts by weight, 30 parts by weight, 31 parts by weight, 32 parts by weight, 33 parts by weight, 34 parts by weight, 35 parts by weight, 36 parts by weight, 37 parts by weight, 38 parts by weight, 39 parts by weight, 40 parts by weight, 41 parts by weight, 42 parts by weight, 43 parts by weight, 44 parts by weight, 45 parts by weight, 46 parts by weight, 47 parts by weight, 48 parts by weight, 49 parts by weight, 50 parts by weight, 51 parts by weight, 52 parts by weight, 53 parts by weight, 54 parts by weight, 55 parts by weight, 56 parts by weight, 57 parts by weight, 58 parts by weight, 59 parts by weight, 60 parts by weight, 61 parts by weight, 62 parts by weight, 63 parts by weight, 64 parts by weight, 65 parts by weight, 66 parts by weight, 67 parts by weight, 68 parts by weight, 69 parts by weight, 70 parts by weight, 71 parts by weight, 72 parts by weight, 73 parts by weight, 74 parts by weight, 75 parts by weight, 76 parts by weight, 77 parts by weight, 78 parts by weight, 79 parts by weight, 80 parts by weight, 81 parts by weight, 82 parts by weight, 83 parts by weight, 84 parts by weight, 85 parts by weight, 86 parts by weight, 87 parts by weight, 88 parts by weight, 89 parts by weight, 90 parts by weight, 91 parts by weight, 92 parts by weight, 93 parts by weight, 94 parts by weight, 95 parts by weight, 96 parts by weight, 97 parts by weight, 98 parts by weight, 99 parts by weight, 100 parts by weight, 101 parts by weight, 102 parts by weight, 103 parts by weight, 104 parts by weight, 105 parts by weight, 106 parts by weight, 107 parts by weight, 108 parts by weight, 109 parts by weight, 110 parts by weight, 111 parts by weight, 112 parts by weight, 113 parts by weight, 114 parts by weight, 115 parts by weight, 116 parts by weight, 117 parts by weight, 118 parts by weight, 119 parts by weight, 120 parts by weight, 121 parts by weight, 122 parts by weight, 123 parts by weight, 124 parts by weight, 125 parts by weight, 126 parts by weight, 127 parts by weight, 128 parts by weight, 129 parts by weight, 130 parts by weight, 131 parts by weight, 132 parts by weight, 133 parts by weight, 134 parts by weight, 135 parts by weight, 136 parts by weight, 137 parts by weight, 138 parts by weight, 139 parts by weight, 140 parts by weight, 141 parts by weight, 142 parts by weight, 143 parts by weight, 144 parts by weight, 145 parts by weight, 146 parts by weight, 147 parts by weight, 148 parts by weight, 149 parts by weight, 150 parts by weight, 151 parts by weight, 152 parts by weight, 153 parts by weight, 154 parts by weight, 155 parts by weight, 156 parts by weight, 157 parts by weight, 158 parts by weight, 159 parts by weight, 160 parts by weight, 161 parts by weight, 162 parts by weight, 163 parts by weight, 164 parts by weight, 165 parts by weight, 166 parts by weight, 167 parts by weight, 168 parts by weight, 169 parts by weight, 170 parts by weight, 171 parts by weight, 172 parts by weight, 173 parts by weight, 174 parts by weight, 175 parts by weight, 176 parts by weight, 177 parts by weight, 178 parts by weight, 179 parts by weight, 180 parts by weight, 181 parts by weight, 182 parts by weight, 183 parts by weight, 184 parts by weight, 185 parts by weight, 186 parts by weight, 187 parts by weight, 188 parts by weight, 189 parts by weight, 190 parts by weight, 191 parts by weight, 192 parts by weight, 193 parts by weight, 194 parts by weight, 195 parts by weight, 196 parts by weight, 197 parts by weight, 198 parts by weight, 199 parts by weight, 200 parts by weight, 201 parts by weight, 202 parts by weight, 203 parts by weight, 204 parts by weight, 205 parts by weight, 206 parts by weight, 207 parts by weight, 208 parts by weight, 209 parts by weight, 210 parts by weight, 211 parts by weight, 212 parts by weight, 213 parts by weight, 214 parts by weight, 215 parts by weight, 216 parts by weight, 217 parts by weight, 218 parts by weight, 219 parts by weight, 220 parts by weight, 221 parts by weight, 222 parts by weight, 223 parts by weight, 224 parts by weight, 225 parts by weight, 226 parts by weight, 227 parts by weight, 228 parts by weight, 229 parts by weight, 230 parts by weight, 231 parts by weight, 232 parts by weight, 233 parts by weight, 234 parts by weight, 235 parts by weight, 236 parts by weight, 237 parts by weight, 238 parts by weight, 239 parts by weight, 240 parts by weight, 241 parts by weight, 242 parts by weight, 243 parts by weight, 244 parts by weight, 245 parts by weight, 246 parts by weight, 247 parts by weight, 248 parts by weight, 249 parts by weight, 250 parts by weight, 251 parts by weight, 252 parts by weight, 253 parts by weight, 254 parts by weight, 255 parts by weight, 256 parts by weight, 257 parts by weight, 258 parts by weight, 259 parts by weight, 260 parts by weight, 261 parts by weight, 262 parts by weight, 263 parts by weight, 264 parts by weight, 265 parts by weight, 266 parts by weight, 267 parts by weight, 268 parts by weight, 269 parts by weight, 270 parts by weight, 271 parts by weight, 272 parts by weight, 273 parts by weight, 274 parts by weight, 275 parts by weight, 276 parts by weight, 277 parts by weight, 278 parts by weight, 279 parts by weight, 280 parts by weight, 281 parts by weight, 282 parts by weight, 283 parts by weight, 284 parts by weight, 285 parts by weight, 286 parts by weight, 287 parts by weight, 288 parts by weight, 289 parts by weight, 290 parts by weight, 291 parts by weight, 292 parts by weight, 293 parts by weight, 294 parts by weight, 295 parts by weight, 296 parts by weight, 297 parts by weight, 298 parts by weight, 299 parts by weight, 300 parts by weight, 301 parts by weight, 302 parts by weight, 303 parts by weight, 304 parts by weight, 305 parts by weight, 306 parts by weight, 307 parts by weight, 308 parts by weight, 309 parts by weight, 310 parts by weight, 311 parts by weight, 312 parts by weight, 313 parts by weight, 314 parts by weight, 315 parts by weight, 316 parts by weight, 317 parts by weight, 318 parts by weight, 319 parts by weight, 320 parts by weight, 321 parts by weight, 322 parts by weight, 323 parts by weight, 324 parts by weight, 325 parts by weight, 326 parts by weight, 327 parts by weight, 328 parts by weight, 329 parts by weight, 330 parts by weight, 331 parts by weight, 332 parts by weight, 333 parts by weight, 334 parts by weight, 335 parts by weight, 336 parts by weight, 337 parts by weight, 338 parts by weight, 339 parts by weight, 340 parts by weight, 341 parts by weight, 342 parts by weight, 343 parts by weight, 344 parts by weight, 345 parts by weight, 346 parts by weight, 347 parts by weight, 348 parts by weight, 349 parts by weight, 350 parts by weight, 351 parts by weight, 352 parts by weight, 353 parts by weight, 354 parts by weight, 355 parts by weight, 356 parts by weight, 357 parts by weight, 358 parts by weight, 359 parts by weight, 360 parts by weight, 361 parts by weight, 362 parts by weight, 363 parts by weight, 364 parts by weight, 365 parts by weight, 366 parts by weight, 367 parts by weight, 368 parts by weight, 369 parts by weight, 370 parts by weight, 371 parts by weight, 372 parts by weight, 373 parts by weight, 374 parts by weight, 375 parts by weight, 376 parts by weight, 377 parts by weight, 378 parts by weight, 379 parts by weight, 380 parts by weight, 381 parts by weight, 382 parts by weight, 383 parts by weight, 384 parts by weight, 385 parts by weight, 386 parts by weight, 387 parts by weight, 388 parts by weight, 389 parts by weight, 390 parts by weight, 391 parts by weight, 392 parts by weight, 393 parts by weight, 394 parts by weight, 395 parts by weight, 396 parts by weight, 397 parts by weight, 398 parts by weight, 399 parts by weight, 400 parts by weight, 401 parts by weight, 402 parts by weight, 403 parts by weight, 404 parts by weight, 405 parts by weight, 406 parts by weight, 407 parts by weight, 408 parts by weight, 409 parts by weight, 410 parts by weight, 411 parts by weight, 412 parts by weight, 413 parts by weight, 414 parts by weight, 415 parts by weight, 416 parts by weight, 417 parts by weight, 418 parts by weight, 419 parts by weight, 420 parts by weight, 421 parts by weight, 422 parts by weight, 423 parts by weight, 424 parts by weight, 425 parts by weight, 426 parts by weight, 427 parts by weight, 428 parts by weight, 429 parts by weight, 430 parts by weight, 431 parts by weight, 432 parts by weight, 433 parts by weight, 434 parts by weight, 435 parts by weight, 436 parts by weight, 437 parts by weight, 438 parts by weight, 439 parts by weight, 440 parts by weight, 441 parts by weight, 442 parts by weight, 443 parts by weight, 444 parts by weight, 445 parts by weight, 446 parts by weight, 447 parts by weight, 448 parts by weight, 449 parts by weight, 450 parts by weight, 451 parts by weight, 452 parts by weight, 453 parts by weight, 454 parts by weight, 455 parts by weight, 456 parts by weight, 457 parts by weight, 458 parts by weight, 459 parts by weight, 460 parts by weight, 461 parts by weight, 462 parts by weight, 463 parts by weight, 464 parts by weight, 465 parts by weight, 466 parts by weight, 467 parts by weight, 468 parts by weight, 469 parts by weight, 470 parts by weight, 471 parts by weight, 472 parts by weight, 473 parts by weight, 474 parts by weight, 475 parts by weight, 476 parts by weight, 477 parts by weight, 478 parts by weight, 479 parts by weight, 480 parts by weight, 481 parts by weight, 482 parts by weight, 483 parts by weight, 484 parts by weight, 485 parts by weight, 486 parts by weight, 487 parts by weight, 488 parts by weight, 489 parts by weight, 490 parts by weight, 491 parts by weight, 492 parts by weight, 493 parts by weight, 494 parts by weight, 495 parts by weight, 496 parts by weight, 497 parts by weight, 498 parts by weight, 499 parts by weight, 500 parts by weight, 501 parts by weight, 502 parts by weight, 503 parts by weight, 504 parts by weight, 505 parts by weight, 506 parts by weight, 507 parts by weight, 508 parts by weight, 509 parts by weight, 510 parts by weight, 511 parts by weight, 512 parts by weight, 513 parts by weight, 514 parts by weight, 515 parts by weight, 516 parts by weight, 517 parts by weight, 518 parts by weight, 519 parts by weight, 520 parts by weight, 521 parts by weight, 522 parts by weight, 523 parts by weight, 524 parts by weight, 525 parts by weight, 526 parts by weight, 527 parts by weight, 528 parts by weight, 529 parts by weight, 530 parts by weight, 531 parts by weight, 532 parts by weight, 533 parts by weight, 534 parts by weight, 535 parts by weight, 536 parts by weight, 537 parts by weight, 538 parts by weight, 539 parts by weight, 540 parts by weight, 541 parts by weight, 542 parts by weight, 543 parts by weight, 544 parts by weight, 545 parts by weight, 546 parts by weight, 547 parts by weight, 548 parts by weight, 549 parts by weight, 550 parts by weight, 551 parts by weight, 552 parts by weight, 553 parts by weight, 554 parts by weight, 555 parts by weight, 556 parts by weight, 557 parts by weight, 558 parts by weight, 559 parts by weight, 560 parts by weight, 561 parts by weight, 562 parts by weight, 563 parts by weight, 564 parts by weight, 565 parts by weight, 566 parts by weight, 567 parts by weight, 568 parts by weight, 569 parts by weight, 570 parts by weight, 571 parts by weight, 572 parts by weight, 573 parts by weight, 574 parts by weight, 575 parts by weight, 576 parts by weight, 577 parts by weight, 578 parts by weight, 579 parts by weight, 580 parts by weight, 581 parts by weight, 582 parts by weight, 583 parts by weight, 584 parts by weight, 585 parts by weight, 586 parts by weight, 587 parts by weight, 588 parts by weight, 589 parts by weight, 590 parts by weight, 591 parts by weight, 592 parts by weight, 593 parts by weight, 594 parts by weight, 595 parts by weight, 596 parts by weight, 597 parts by weight, 598 parts by weight, 599 parts by weight, 600 parts by weight, 601 parts by weight, 602 parts by weight, 603 parts by weight, 604 parts by weight, 605 parts by weight, 606 parts by weight, 607 parts by weight, 608 parts by weight, 609 parts by weight, 610 parts by weight, 611 parts by weight, 612 parts by weight, 613 parts by weight, 614 parts by weight, 615 parts by weight, 616 parts by weight, 617 parts by weight, 618 parts by weight, 619 parts by weight, 620 parts by weight, 621 parts by weight, 622 parts by weight, 623 parts by weight, 624 parts by weight, 625 parts by weight, 626 parts by weight, 627 parts by weight, 628 parts by weight, 629 parts by weight, 630 parts by weight, 631 parts by weight, 632 parts by weight, 633 parts by weight, 634 parts by weight, 635 parts by weight, 636 parts by weight, 637 parts by weight, 638 parts by weight, 639 parts by weight, 640 parts by weight, 641 parts by weight, 642 parts by weight, 643 parts by weight, 644 parts by weight, 645 parts by weight, 646 parts by weight, 647 parts by weight, 648 parts by weight, 649 parts by weight, 650 parts by weight, 651 parts by weight, 652 parts by weight, 653 parts by weight, 654 parts by weight, 655 parts by weight, 656 parts by weight, 657 parts by weight, 658 parts by weight, 659 parts by weight, 660 parts by weight, 661 parts by weight, 662 parts by weight, 663 parts by weight, 664 parts by weight, 665 parts by weight, 666 parts by weight, 667 parts by weight, 668 parts by weight, 669 parts by weight, 670 parts by weight, 671 parts by weight, 672 parts by weight, 673 parts by weight, 674 parts by weight, 675 parts by weight, 676 parts by weight, 677 parts by weight, 678 parts by weight, 679 parts by weight, 680 parts by weight, 681 parts by weight, 682 parts by weight, 683 parts by weight, 684 parts by weight, 685 parts by weight, 686 parts by weight, 687 parts by weight, 688 parts by weight, 689 parts by weight, 690 parts by weight, 691 parts by weight, 692 parts by weight, 693 parts by weight, 694 parts by weight, 695 parts by weight, 696 parts by weight, 697 parts by weight, 698 parts by weight, 699 parts by weight, 700 parts by weight, 701 parts by weight, 702 parts by weight, 703 parts by weight, 704 parts by weight, 705 parts by weight, 706 parts by weight, 707 parts by weight, 708 parts by weight, 709 parts by weight, 710 parts by weight, 711 parts by weight, 712 parts by weight, 713 parts by weight, 714 parts by weight, 715 parts by weight, 716 parts by weight, 717 parts by weight, 718 parts by weight, 719 parts by weight, 720 parts by weight, 721 parts by weight, 722 parts by weight, 723 parts by weight, 724 parts by weight, 725 parts by weight, 726 parts by weight, 727 parts by weight, 728 parts by weight, 729 parts by weight, 730 parts by weight, 731 parts by weight, 732 parts by weight, 733 parts by weight, 734 parts by weight, 735 parts by weight, 736 parts by weight, 737 parts by weight, 738 parts by weight, 739 parts by weight, 740 parts by weight, 741 parts by weight, 742 parts by weight, 743 parts by weight, 744 parts by weight, 745 parts by weight, 746 parts by weight, 747 parts by weight, 748 parts by weight, 749 parts by weight, 750 parts by weight, 751 parts by weight, 752 parts by weight, 753 parts by weight, 754 parts by weight, 755 parts by weight, 756 parts by weight, 757 parts by weight, 758 parts by weight, 759 parts by weight, 760 parts by weight, 761 parts by weight, 762 parts by weight, 763 parts by weight, 764 parts by weight, 765 parts by weight, 766 parts by weight, 767 parts by weight, 768 parts by weight, 769 parts by weight, 770 parts by weight, 771 parts by weight, 772 parts by weight, 773 parts by weight, 774 parts by weight, 775 parts by weight, 776 parts by weight, 777 parts by weight, 778 parts by weight, 779 parts by weight, 780 parts by weight, 781 parts by weight, 782 parts by weight, 783 parts by weight, 784 parts by weight, 785 parts by weight, 786 parts by weight, 787 parts by weight, 788 parts by weight, 789 parts by weight, 790 parts by weight, 791 parts by weight, 792 parts by weight, 793 parts by weight, 794 parts by weight, 795 parts by weight, 796 parts by weight, 797 parts by weight, 798 parts by weight, 799 parts by weight, 800 parts by weight, etc, and other specific point values within this numerical range or the values between any two points thereof can be selected and will not be described redundantly here.

In one embodiment, the present invention provides an oral polypeptide composition comprising a polypeptide, fish oil, a protease inhibitor, an absorption enhancer and a surfactant, wherein, when the polypeptide is present in the composition in an amount of 1 part by weight,

the fish oil is present in the composition in an amount of 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.5, 2, 2.5, 3.0, 3.5, 4.0, 4.5, 5.0, 5.5, 6.0, 6.5, 7.0, 7.5, 8.0, 8.5, 9.0, 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800 parts by weight or the values between any two points thereof;
the protease inhibitor is present in the composition in an amount of 0.1, 0.2, 0.3, 0.4, 0.5, 0.6, 0.7, 0.8, 0.9, 1.0, 1.5, 2, 2.5, 3.0, 3.5, 4.0, 4.5, 5.0, 5.5, 6.0, 6.5, 7.0, 7.5, 8.0, 8.5, 9.0, 10, 15, 20, 25, 30, 35, 40, 45, 50, 55, 60, 65, 70, 75, 80, 85, 90, 95, 100, 110, 120, 130, 140, 150, 160, 170, 180, 190, 200, 210 parts by weight or the values between any two points thereof;
the absorption enhancer is present in the composition in an amount of 0.1, 0.2, 0.3, 0.4, 0.5, 0.6, 0.7, 0.8, 0.9, 1.0, 1.5, 2, 2.5, 3.0, 3.5, 4.0, 4.5, 5.0, 5.5, 6.0, 6.5, 7.0, 7.5, 8.0, 8.5, 9.0, 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300 parts by weight or the values between any two points thereof; and
the surfactant is present in the composition in an amount of 1.0, 1.5, 2, 2.5, 3.0, 3.5, 4.0, 4.5, 5.0, 5.5, 6.0, 6.5, 7.0, 7.5, 8.0, 8.5, 9.0, 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800 parts by weight or the values between any two points thereof.

In one embodiment, the present invention provides an oral polypeptide composition comprising a polypeptide, fish oil, a protease inhibitor, an absorption enhancer and a surfactant, wherein, when the polypeptide is present in the composition in an amount of 1 part by weight,

the fish oil is present in the composition in an amount of 0.01 to 800 parts by weight, for example, preferably 0.02 to 790 parts by weight, 0.03 to 780 parts by weight, 0.04 to 770 parts by weight, 0.05 to 760 parts by weight, 0.06 to 750 parts by weight, 0.07 to 740 parts by weight, 0.08 to 730 parts by weight, 0.09 to 720 parts by weight, 0.1 to 710 parts by weight, 0.2 to 700 parts by weight, 0.3 to 690 parts by weight, 0.4 to 680 parts by weight, 0.5 to 670 parts by weight, 0.6 to 660 parts by weight, 0.7 to 650 parts by weight, 0.8 to 640 parts by weight, 0.9 to 630 parts by weight, 1.0 to 620 parts by weight, 1.5 to 610 parts by weight, 2 to 600 parts by weight, 2.5 to 590 parts by weight, 3.0 to 580 parts by weight, 3.5 to 570 parts by weight, 4.0 to 560 parts by weight, 4.5 to 550 parts by weight, 5.0 to 540 parts by weight, 5.5 to 530 parts by weight, 6.0 to 520 parts by weight, 6.5 to 510 parts by weight, 7.0 to 500 parts by weight, 7.5 to 490 parts by weight or 8.0 to 480 parts by weight;
the protease inhibitor is present in the composition in an amount of 0.1 to 210 parts by weight, for example, preferably 0.2 to 200 parts by weight, 0.3 to 190 parts by weight, 0.4 to 180 parts by weight, 0.5 to 170 parts by weight, 0.6 to 160 parts by weight, 0.7 to 150 parts by weight, 0.8 to 140 parts by weight, 0.9 to 130 parts by weight, 1.0 to 120 parts by weight, 1.5 to 110 parts by weight, 2 to 100 parts by weight, 2.5 to 90 parts by weight or 3.0 to 80 parts by weight;
the absorption enhancer is present in the composition in an amount of 0.1 to 300 parts by weight, for example, preferably 0.2 to 290 parts by weight, 0.3 to 280 parts by weight, 0.4 to 270 parts by weight, 0.5 to 260 parts by weight, 0.6 to 250 parts by weight, 0.7 to 240 parts by weight, 0.8 to 230 parts by weight, 0.9 to 220 parts by weight, 1.0 to 210 parts by weight, 1.5 to 200 parts by weight or 2 to 190 parts by weight; and
the surfactant is present in the composition in an amount of 0.01 to 800 parts by weight, for example, preferably 0.02 to 790 parts by weight, 0.03 to 780 parts by weight, 0.04 to 770 parts by weight, 0.05 to 760 parts by weight, 0.06 to 750 parts by weight, 0.07 to 740 parts by weight, 0.08 to 730 parts by weight, 0.09 to 720 parts by weight, 0.1 to 710 parts by weight, 0.2 to 700 parts by weight, 0.3 to 690 parts by weight, 0.4 to 680 parts by weight, 0.5 to 670 parts by weight, 0.6 to 660 parts by weight, 0.7 to 650 parts by weight, 0.8 to 640 parts by weight, 0.9 to 630 parts by weight, 1.0 to 620 parts by weight, 1.5 to 610 parts by weight, 2 to 600 parts by weight, 2.5 to 590 parts by weight, 3.0 to 580 parts by weight, 3.5 to 570 parts by weight, 4.0 to 560 parts by weight, 4.5 to 550 parts by weight, 5.0 to 540 parts by weight, 5.5 to 530 parts by weight, 6.0 to 520 parts by weight, 6.5 to 510 parts by weight, 7.0 to 500 parts by weight, 7.5 to 490 parts by weight or 8.0 to 480 parts by weight.

In one embodiment of the present invention, the oral polypeptide composition has a content of poypeptide of >85% after being stored under suitable conditions for a period of time. For example, the oral polypeptide composition has a content of poypeptide of >85%, >86%, >87%, >88%, >89%, >90%, >91%, >92%, >93%, >94%, >95%, >96%, >97%, >98%, >99% after being stored at 2°C to 8°C for 15 months; the oral polypeptide composition has a content of poypeptide of >85%, >86%, >87%, >88%, >89%, >90%, >91%, >92%, >93%, >94%, >95%, >96%, >97%, >98%, >99% after being stored at 2°C to 8°C for 18 months; the oral polypeptide composition has a content of poypeptide of >85%, >86%, >87%, >88%, >89%, >90%, >91%, >92%, >93%, >94%, >95%, >96%, >97%, >98%, >99% after being stored at 2°C to 8°C for 24 months; the oral polypeptide composition has a content of poypeptide of >85%, >86%, >87%, >88%, >89%, >90%, >91%, >92%, >93%, >94%, >95%, >96%, >97%, >98%, >99% after being stored at 2°C to 8°C for 30 months; the oral polypeptide composition has a content of poypeptide of >85%, >86%, >87%, >88%, >89%, >90%, >91%, >92%, >93%, >94%, >95%, >96%, >97%, >98%, >99% after being stored at 2°C to 8°C for 36 months.

In one embodiment of the present invention, the oral polypeptide composition has a content of poypeptide of >85%, >90%, >91%, >95%, >98% after being stored at 25°C for 5 days; has a content of poypeptide of >70%, >75%, >80%, >85%, 90% after being stored at 25°C for 10 days; has a content of poypeptide of >80% after being stored at 25°C for 30 days; and has a content of poypeptide of >50%, >60%, >70%, >75% after being stored for 60 days.

In one embodiment of the present invention, the oral polypeptide composition has a content of poypeptide of >70%, >80% after being stored at 40°C for 5 days; and has a content of poypeptide of >60%, >70%, >80% after being stored at 40°C for 10 days.

In one embodiment of the present invention, the cumulative dissolution of the polypeptide in the polypeptide composition is significantly higher than that of the prior art.

In one embodiment of the present invention, the cumulative dissolution of the soybean trypsin inhibitor in the polypeptide composition is significantly higher than that of the prior art.

In one embodiment of the present invention, using phosphate buffer solution at pH 6.8 as a dissolution medium, the oral polypeptide composition has a polypeptide cumulative dissolution of >30%, >35%, >40%, >45%, >50%, >55%, >60%, >65%, >70%, >75%, >80%, >85%, >90% within 2 minutes; >35%, >40%, >45%, >50%, >55%, >60%, >65%, >70%, >75%, >80%, >85%, >90%, >95%, >96%, >97%, >98% within 5 minutes; >40%, >45%, >50%, >55%, >60%, >65%, >70%, >75%, >80%, >85%, >90%, >95%, >96%, >97%, >98% within 10 minutes; >60%, >65%, >70%, >75%, >80%, >85%, >90%, >95%, >96%, >97%, >98% within 20 minutes; >80%, >85%, >90%, >95%, >96%, >97%, >98% within 30 minutes; >90%, >95%, >96%, >97%, >98% within 45 minutes; and >90%, >95%, >96%, >97%, >98% within 60 minutes.

In one embodiment of the present invention, using phosphate buffer solution at pH 6.8 as a dissolution medium, the oral polypeptide composition has a soybean trypsin inhibitor cumulative dissolution of >40%, >45%, >50%, >55%, >60%, >65%, >70%, >75%, >80%, >85%, >90% within 2 minutes; >15%, >20%, >30%, 35%, >40%, >45%, >50%, >55%, >60%, >65%, >70%, >75%, >80%, >85%, >90%, >95%, >96%, >97%, >98% within 5 minutes; >15%, >20%, >30%, 35%, 40%, >45%, >50%, >55%, >60%, >65%, >70%, >75%, >80%, >85%, >90%, >95%, >96%, >97%, >98% within 10 minutes; >20%, >30%, 35%, 40%, >45%, >50%, >55%, >60%, >65%, >70%, >75%, >80%, >85%, >90%, >95%, >96%, >97%, >98% within 20 minutes; >40%, >45%, >50%, >55%, >60%, >65%, >70%, >75%, >80%, >85%, >90%, >95%, >96%, >97%, >98% within 30 minutes; >50%, >55%, >60%, >65%, >70%, >75%, >80%, >85%, >90%, >95%, >96%, >97%, >98% within 45 minutes; >50%, >55%, >60%, >65%, >70%, >75%, >80%, >85%, >90%, >95%, >96%, >97%, >98% within 60 minutes.

The measurement of the cumulative dissolution is influenced by various factors, and it is inevitable that there may be a certain deviation in the data. The cumulative dissolution in the present invention can have an error range of ± 5%.

In the context of the polypeptide stability, the term "content of polypeptide" used herein refers to the percentage of the actual content of the polypeptide relative to the labeled amount. For example, for a capsule formulation of Liraglutide, the content of Liraglutide in the capsule refers to $\frac{the\ actual\ content\ of\ Liraglutide\ in\ each\ capsule}{the\ labeled\ amount\ of\ Liraglutide\ in\ each\ capsule} \times 100\%$. For example, if a prescription for a capsule indicates that it contains 20 mg of Liraglutide, it is sampled and tested, and the test results show that each capsule actually contains 18 mg of Liraglutide, the content of Liraglutide is $\frac{18\ mg}{20\ mg} \times 100\% = 90\%$.

In addition, the oral polypeptide composition increases the bioavailability of polypeptide in a subject by at least 5%, at least 8%, at least 10%, at least 15%, at least 18%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, at least 160%, at least 170%, at least 200%, at least 300%, at least 400%, at least 500%.

In a preferable technical solution of the present invention, the polypeptide in the oral polypeptide composition has a molecular weight of 100 Da to 20000 Da, and for example, may have a molecular weight of 100 Da, 200 Da, 300 Da, 400 Da, 500 Da, 600 Da, 700 Da, 800 Da, 900 Da, 1000 Da, 1100 Da, 1200 Da, 1300 Da, 1400 Da, 1500 Da, 1600 Da, 1700 Da, 1800 Da, 1900 Da, 2000 Da, 2100 Da, 2200 Da, 2300 Da, 2400 Da, 2500 Da, 2600 Da, 2700 Da, 2800 Da, 2900 Da, 3000 Da, 3100 Da, 3200 Da, 3300 Da, 3400 Da, 3500 Da, 3600 Da, 3700 Da, 3800 Da, 3900 Da, 4000 Da, 4100 Da, 4200 Da, 4300 Da, 4400 Da, 4500 Da, 4600 Da, 4700 Da, 4800 Da, 4900 Da, 5000 Da, 5100 Da, 5200 Da, 5300 Da, 5400 Da, 5500 Da, 5600 Da, 5700 Da, 5800 Da, 5900 Da, 6000 Da, 6100 Da, 6200 Da, 6300 Da, 6400 Da, 6500 Da, 6600 Da, 6700 Da, 6800 Da, 6900 Da, 7000 Da, 7100 Da, 7200 Da, 7300 Da, 7400 Da, 7500 Da, 7600 Da, 7700 Da, 7800 Da, 7900 Da, 8000 Da, 8100 Da, 8200 Da, 8300 Da, 8400 Da, 8500 Da, 8600 Da, 8700 Da, 8800 Da, 8900 Da, 9000 Da, 9100 Da, 9200 Da, 9300 Da, 9400 Da, 9500 Da, 9600 Da, 9700 Da, 9800 Da, 9900 Da, 10000 Da, 11000 Da, 12000 Da, 13000 Da, 14000 Da, 15000 Da, 16000 Da, 17000 Da, 18000 Da, 19000 Da, 20000 Da, or any range between the above values, preferably 1000 Da to 20000 Da, preferably 1000 Da to 6000 Da, and further preferably 2000 Da to 6000 Da.

In one embodiment of the oral polypeptide composition according to the present invention, the polypeptide is any one or more selected from the group consisting of insulin or derivatives or analogues thereof, glucagon, glucagon-like peptide-1 receptor agonist, glucagon-like peptide-2 receptor agonist, calcitonin, growth hormone, somatostatin, thyrotropin-releasing hormone, parathyroid hormone, gonadotropin-releasing hormone, heparin, granulocyte colony-stimulating factor, prostaglandin, cyclosporin, interferon, vasopressin, vancomycin, erythrogenin, glutathione and thymosin;

Preferably, the polypeptide is any one or more selected from the group consisting of animal insulin, human insulin, recombinant human insulin, insulin derivatives, insulin analogues, glucagon, Exenatide, Benaglutide, Liraglutide, Loxenatide, Dulaglutide, Lixisenatide, Semaglutide, Albiglutide, Dulaglutide, Teduglutide, Lixisenatide, calcitonin, salmon calcitonin, growth hormone, Octreotide, thyrotropin-releasing hormone, parathyroid hormone fragment, Teriparatide, Abaloparatide, Leuprorelin, Protirelin, Gonadorelin, Goserelin, Buserelin, Sermorelin, Nafarelin, Histrelin, Triptorelin, Tesamorelin, Corticorelin, heparin, granulocyte colony-stimulating factor, prostaglandin, cyclosporin, interferon, vasopressin, vancomycin, erythrogenin, Lanreotide Acetate, Terlipressin, Antiangiotide, Nesiritide, Eptifibatide, Sifuvirtide, Atosiban, Ossotide, Muscular Amino Acids and Peptides and Nucleosides, glutathione, mannatide, Enfuvirtide, bradykinin, enkephalin, Nosiheptide, hirudin, Glatiramer acetate, aprotinin, Alarelin, pancreatic kininogenase, polymyxin, Serrapeptase, thymosin $\alpha$1, thymopentin and thymalfasin.

Preferably, the polypeptide is any one or more selected from the group consisting of swine insulin, bovine insulin, human insulin, recombinant human insulin, Insulin Lispro, Insulin Aspart, isophane insulin, protamine zinc insulin, Insulin Glargine, Insulin Detemir, Insulin Degludec, glucagon, Exenatide, Benaglutide, Liraglutide, Loxenatide, Dulaglutide, Lixisenatide, Semaglutide, Albiglutide, Dulaglutide, Teduglutide, Lixisenatide, calcitonin, salmon calcitonin, recombinant growth hormone, Octreotide, thyrotropin-releasing hormone, parathyroid hormone fragment, Teriparatide, Abaloparatide, Leuprorelin, Protirelin, Gonadorelin, Goserelin, Buserelin, Sermorelin, Nafarelin, Histrelin, Triptorelin, Tesamorelin, Corticorelin, heparin, granulocyte colony-stimulating factor, prostaglandin, ciclosporin, recombinant human interferon $\alpha$2b, recombinant human interferon b, recombinant human interferon $\gamma$, vasopressin, vancomycin, erythrogenin, Lanreotide Acetate, Terlipressin, Antiangiotide, Nesiritide, Eptifibatide, Sifuvirtide, Atosiban, Ossotide, Muscular Amino Acids and Peptides and Nucleosides, glutathione, mannatide, Enfuvirtide, bradykinin, enkephalin, Nosiheptide, hirudin, Glatiramer acetate, aprotinin, Alarelin, pancreatic kininogenase, polymyxin, Serrapeptase, thymosin $\alpha$1, thymopentin and thymalfasin.

The insulin in the present invention includes animal insulin, human insulin, recombinant human insulin, proinsulin, and insulin-like compounds including insulin analogues and insulin derivatives and the like. The insulin in the present invention includes rapid-acting insulins, short-acting insulins, intermediate-acting insulins, long-acting insulins, and ultra-long-acting insulins.

The insulin analogues in the present invention refer to insulin in which one or more amino acids are substituted while maintaining some or all of the glucose-related activities of insulin.

The insulin derivatives in the present invention generally refer to compounds that can be prepared from natural peptides or analogues thereof by chemical modification, particularly by covalent linkage of one or more substituents.

The insulin, proinsulin, insulin analogues, and insulin derivatives in the present invention include: (1) rapid-acting insulins (sometimes also referred to as "monomeric insulin analogues"), such as Insulin Lispro and Insulin Aspart; (2) short-acting insulins (sometimes also referred to as "conventional" insulins); (3) intermediate-acting insulin, such as isophane insulin (NPH) and recombinant human insulin; (4) long-acting insulins (so-called "basal insulins"), such as protamine zinc insulin, Insulin Glargine and Insulin Detemir; (5) utra-long-acting insulins; (6) position-shifted insulin analogues; (7) insulin deletion analogues; (8) derived insulin; (9) derived insulin analogues; (10) derived proinsulin; (11) human insulin analogue complexes (such as hexamer complexes), (12) insulin mixtures, and (13) PEG insulin.

The glucagon-like peptide-1 in the present invention includes short-acting and long-acting GLP-1 receptor agonist.

The glucagon-like peptide-1 includes Exenatide, Lixisenatide, Benaglutide, Liraglutide, Taspoglutid, Loxenatide, Semaglutide, Albiglutide, Dulaglutide and the like.

The thymosin in the present invention includes thymosin $\alpha$1, thymalfasin, thymosin $\beta$4, or thymopentin. It should be noted that, the polypeptide in the composition of the present invention may also be replaced with other active substances, such as other oral small-molecule substances with poor bioavailability.

In a preferable technical solution of the present invention, the protease inhibitor targets a target protein which is any one or more selected from the group consisting of serine protease, trypsin, chymotrypsin, carboxypeptidase and aminopeptidase;

preferably, the protease inhibitor is any one or more selected from the group consisting of cysteine protease inhibitor, serine protease inhibitor, trypsin inhibitor, threonine protease inhibitor, aspartic protease inhibitor and metalloproteinase inhibitor;

preferably, the protease inhibitor is any one or more selected from the group consisting of soybean trypsin inhibitor, 4-(2-aminoethyl)benzenesulfonylfluoride hydrochloride, $\varepsilon$-aminocaproic acid, antiprotease, $\alpha$1-antichymotrypsin, antithrombin, $\alpha$1-antitrypsin, 4-amidinophenyl-methanesulfonyl fluoride, aprotinin, benzamidine-HCl, chymotrypsin, diisopropylfluoro-phosphate, leupeptin, phenylmethylsulfonyl fluoride, 1-chloro-3-sulfonylamino-7-amino-2-heptanone HCl, 1-chloro-3-sulfonylamino-4-phenyl-2-butanone, pentamidine isethionate, egg white trypsin inhibitor, pepstatin, $\alpha$2-macroglobulin, guanidinium, iodoacetic acid, zinc and chelating agents of zinc.

In one embodiment of the present invention, the soybean trypsin inhibitor is selected from the group consisting of Kunitz trypsin inhibitor and/or Bowman-Birk trypsin inhibitor.

In one embodiment of the present invention, the at least one absorption enhancer is any one or more selected from the group consisting of ethylenediamine tetraacetic acid or salts thereof, N-(8-(2-hydroxybenzoyl)amino)octanoic acid or salts thereof, salicylic acid or salts thereof, citric acid or salts thereof, cholic acid or salts thereof, deoxycholic acid or salts thereof, glycocholic acid or salts thereof, taurocholic acid or salts thereof, alkyl glycosides, sodium dodecyl sulfate, docusate sodium, cyclodextrin or derivatives thereof, chitosan or derivatives thereof, octanoic acid or salts thereof, decanoic acid or salts thereof, lauric acid or salts thereof, myristic acid, palmitic acid, stearic acid, fatty acid triglycerides, lecithins, choline and carnitine, and preferably, the salt of ethylenediamine tetraacetic acid is selected from disodium ethylenediaminetetraacetate, dipotassium ethylenediaminetetraacetate, trisodium ethylenediaminetetraacetate or tetrasodium ethylenediaminetetraacetate.

In one embodiment of the present invention, the at least one surfactant is any one or more selected from the group consisting of ionic surfactant, nonionic surfactant and amphoteric surfactant;

preferably, the at least one absorption enhancer is any one or more selected from the group consisting of ammonium dodecyl sulfate, N-dodecyl-$\beta$-D-maltoside, tridecyl-$\beta$-D-maltoside, sodium dodecyl sulfate, docusate sodium, cyclodextrin or derivatives thereof, chitosan or derivatives thereof, sodium polyoxyethylene alkyl sulfate, sodium laureth sulfate, sodium dioctyl sulfosuccinate, poloxamer, glyceryl monostearate, glycerol monolaurate, sorbitan monolaurate, sorbitan monostearate, polyoxyethylene sorbitan fatty acid esters, sorbitan fatty acid esters, polyoxyethylene monopalmitate, polyoxyethylene hydrogenated castor oil, $C_6$-$C_{12}$ fatty acid triglycerides and $C_8$-$C_{10}$ fatty acid triglycerides.

Preferably, the at least one surfactant is selected from the group consisting of Tween, and preferably, the at least one surfactant is any one or more selected from the group consisting of Tween 20, Tween 40, Tween 60, Tween 65, Tween 80 and Tween 85.

In one embodiment of the present invention, by controlling the weight ratio of the fish oil to the surfactant in the oral polypeptide composition, the cumulative dissolution of the polypeptide in the oral polypeptide composition is significantly increased.

In one embodiment of the present invention, by controlling the weight ratio of the fish oil to the surfactant in the oral polypeptide composition, the cumulative dissolution of the soybean trypsin inhibitor in the oral polypeptide composition is significantly increased.

In one embodiment of the present invention, the weight ratio of the fish oil to the surfactant is 1: (0.2 to 15), preferably 1: (0.43 to 9), preferably 1: (0.43 to 5), preferably 1: (0.43 to 3), preferably 1: (0.43 to 2.33), preferably 1: (0.43 to 1), preferably 1:0.5 to 1:2, preferably 1: 0.8 to 1:1.2, or any values between any two ratios above, and preferably 1:0.8 to 1:1.2.

In one embodiment, the present invention provides an oral polypeptide composition, wherein the weight ratio of the fish oil to the surfactant is 1:1, or 1:2.33, or 1:0.43, or 1:9.

In one embodiment, the present invention provides an oral polypeptide composition, wherein the polypeptide is human insulin, the protease inhibitor is soybean trypsin inhibitor, the absorption enhancer is selected from ethylenediamine tetraacetic acid or salts thereof, and the surfactant is selected from Tween.

In one embodiment, the present invention provides an oral polypeptide composition, wherein the polypeptide is recombinant human insulin, the protease inhibitor is soybean trypsin inhibitor, the absorption enhancer is ethylenediamine tetraacetic acid or disodium ethylenediaminetetraacetate, and the surfactant is Tween 80.

In one embodiment of the present invention, by adjusting the weight ratio of the fish oil to the surfactant, the stability and bioavailability of the oral polypeptide composition obtained are improved.

As used herein, the term "fish oil", also known as $\omega$-3 fatty acid triglycerides or Omega-3-acid triglycerides, is a type of oil extracted from a fatty fish, which is rich in various n-3 polyunsaturated fatty acids such as eicosapentaenoic acid (EPA) and docosahexaenoic acid (DHA). The fatty fish useful for the extraction of fish oil includes, but is not limited to: mackerel, tuna, salmon, sturgeon, anchovy, sardine, herring and trout.

In one embodiment of the present invention, the present invention also provides a medicine comprising the oral polypeptide composition as described above.

In one embodiment of the present invention, the medicine further comprises a pharmaceutically acceptable auxiliary material.

Preferably, the pharmaceutically acceptable auxiliary material includes a coating that is any one or more selected from the group consisting of hydroxypropyl cellulose, hydroxypropyl methylcellulose, acrylic resin, polyvinylpyrrolidone, cellulose acetate phthalate, hydroxypropyl methylcellulose phthalate and hydroxypropyl methylcellulose acetate succinate. Preferably, the pharmaceutically acceptable auxiliary material further includes any one or more of an excipient, a disintegrant, a binder, a flavoring agent, and a lubricant.

It should be specified that, the coating in the present disclosure mainly functions to release polypeptide in a specific part of the gastrointestinal tract. For example, enteric soluble coatings can make polypeptide insoluble in gastric acid but soluble in intestinal fluid. Coating materials can be selected by those skilled in the art according to actual requirements, enabling polypeptide to be released in gastric or intestinal fluid in the gastrointestinal tract.

Preferably, the excipient is any one or more selected from the group consisting of lactose, sorbitol, xylitol, mannitol, calcium sulfate, calcium carbonate, calcium hydrogen phosphate, microcrystalline cellulose, silicified microcrystalline cellulose, starch, pregelatinized starch, lactose starch complex, lactose cellulose complex, and mannitol starch complex.

Preferably, the disintegrant is any one or more selected from the group consisting of cross-linked sodium carboxymethyl cellulose, cross-linked polyvinylpyrrolidone, sodium carboxymethyl starch, cross-linked sodium carboxymethyl starch, and low-substituted hydroxypropyl cellulose.

Preferably, the binder is any one or more selected from the group consisting of starch slurry, gelatin solution, sucrose solution, methyl cellulose, ethyl cellulose, sodium carboxymethyl cellulose, hydroxypropyl methylcellulose, hydroxypropyl cellulose, and polyvinylpyrrolidone.

Preferably, the lubricant is any one or more selected from the group consisting of sodium stearyl fumarate, magnesium lauryl sulfate, polyethylene glycol, magnesium dodecyl sulfate, sodium dodecyl sulfate, magnesium stearate, calcium stearate, micronized silica, and talc powder.

Preferably, the flavoring agent is any one or more selected from the group consisting of mannitol, sorbitol, aspartame, stevioside, sucralose, aspartame, neotame, steviosin, sucralose and flavors.

In one embodiment of the present invention, the dosage form of the medicine is any one or more selected from the group consisting of a tablet, a pill, a capsule, an emulsion, a syrup or a suspension.

In one embodiment of the present invention, the oral polypeptide composition or medicine is anhydrous.

In one embodiment of the present invention, the oral polypeptide composition is in the form of a capsule of which the content comprises recombinant human insulin, fish oil, soybean trypsin inhibitor, disodium ethylenediaminetetraacetate, and Tween 80. The capsule content is a suspension that is anhydrous. The term "anhydrous" means that, no water is intentionally added to the capsule content during the process of preparing the oral polypeptide composition of the present invention, but it does not exclude that individual components of the capsule content may absorb moisture from the air.

In one embodiment of the present invention, the system consisting of a polypeptide, fish oil, a protease inhibitor, an absorption enhancer and a surfactant is a suspension.

In one embodiment of the present invention, the system consisting of a polypeptide, fish oil, a protease inhibitor, an absorption enhancer, a surfactant and other pharmaceutically acceptable auxiliary materials is a suspension.

In one embodiment of the present invention, the system consisting of a polypeptide, fish oil, a protease inhibitor, an absorption enhancer and a surfactant is a non-nano system.

In one embodiment of the present invention, the system consisting of a polypeptide, fish oil, a protease inhibitor, an absorption enhancer, a surfactant and other pharmaceutically acceptable auxiliary materials is a non-nano system.

In one embodiment of the present invention, the system consisting of a polypeptide, fish oil, a protease inhibitor, an absorption enhancer and a surfactant is not an emulsion such as oil-in-water or water-in-oil emulsion.

In one embodiment of the present invention, the system consisting of a polypeptide, fish oil, a protease inhibitor, an absorption enhancer, a surfactant and other pharmaceutically acceptable auxiliary materials is not an emulsion such as oil-in-water or water-in-oil emulsion.

In one embodiment of the present invention, the system consisting of a polypeptide, fish oil, a protease inhibitor, an absorption enhancer and a surfactant is not a multiple emulsion such as water-in-oil-in-water or oil-in-water-in-oil emulsion.

In one embodiment of the present invention, the system consisting of a polypeptide, fish oil, a protease inhibitor, an absorption enhancer, a surfactant and other pharmaceutically acceptable auxiliary materials is not a multiple emulsion such as water-in-oil-in-water or oil-in-water-in-oil emulsion.

In one embodiment of the present invention, the oral polypeptide composition is absorbed via the intestinal tract.

In one embodiment of the present invention, the present invention provides a pharmaceutical composition comprising two or more different polypeptides, fish oil, a protease inhibitor, at least one absorption enhancer and at least one surfactant, wherein

the two or more different polypeptides are present in a total amount of 0.1 to 100 parts by weight;

the fish oil is present in an amount of 0.01 to 800 parts by weight;

the protease inhibitor is present in an amount of 0.1 to 210 parts by weight;

the absorption enhancer(s) is/are present in an amount of 0.1 to 300 parts by weight; and

the surfactant(s) is/are present in an amount of 1 to 800 parts by weight;

preferably,

the two or more different polypeptides are present in a total amount of 1 part by weight;

the fish oil is present in an amount of 0.01 to 800 parts by weight;

the protease inhibitor is present in an amount of 0.1 to 210 parts by weight;

the absorption enhancer(s) is/are present in an amount of 0.1 to 300 parts by weight; and

the surfactant(s) is/are present in an amount of 1 to 800 parts by weight;

preferably,

the two or more different polypeptides are present in the same formulation, or independently present in different formulations;

preferably, the two or more different polypeptides are any two or more selected from the group consisting of insulin or derivatives or analogues thereof, glucagon, glucagon-like peptide-1 receptor agonist, glucagon-like peptide-2 receptor agonist, calcitonin, growth hormone, somatostatin, thyrotropin-releasing hormone, parathyroid hormone, gonadotropin-releasing hormone, heparin, granulocyte colony-stimulating factor, prostaglandin, cyclosporin, interferon, vasopressin, vancomycin, erythrogenin, glutathione and thymosin;

preferably, the two or more different polypeptides are any two or more selected from the group consisting of animal insulin, human insulin, recombinant human insulin, insulin derivatives, insulin analogues, glucagon, Exenatide, Benaglutide, Liraglutide, Loxenatide, Dulaglutide, Lixisenatide, Semaglutide, Albiglutide, Dulaglutide, Teduglutide, Lixisenatide, calcitonin, salmon calcitonin, growth hormone, Octreotide, thyrotropin-releasing hormone, parathyroid hormone fragment, Teriparatide, Abaloparatide, Leuprorelin, Protirelin, Gonadorelin, Goserelin, Buserelin, Sermorelin, Nafarelin, Histrelin, Triptorelin, Tesamorelin, Corticorelin, heparin, granulocyte colony-stimulating factor, prostaglandin, cyclosporin, interferon, vasopressin, vancomycin, erythrogenin, Lanreotide Acetate, Terlipressin, Antiangiotide, Nesiritide, Eptifibatide, Sifuvirtide, Atosiban, Ossotide, Muscular Amino Acids and Peptides and Nucleosides, glutathione, mannatide, Enfuvirtide, bradykinin, enkephalin, Nosiheptide, hirudin, Glatiramer acetate, aprotinin, Alarelin, pancreatic kininogenase, polymyxin, Serrapeptase, thymosin $\alpha 1$, thymopentin and thymalfasin;

preferably, the polypeptides are any two or more selected from the group consisting of swine insulin, bovine insulin, human insulin, recombinant human insulin, Insulin Lispro, Insulin Aspart, isophane insulin, protamine zinc insulin, Insulin Glargine, Insulin Detemir, Insulin Degludec, glucagon, Exenatide, Benaglutide, Liraglutide, Loxenatide, Dulaglutide, Lixisenatide, Semaglutide, Albiglutide, Dulaglutide, Teduglutide, Lixisenatide, calcitonin, salmon calcitonin, recombinant growth hormone, Octreotide, thyrotropin-releasing hormone, parathyroid hormone fragment, Teriparatide, Abaloparatide, Leuprorelin, Protirelin, Gonadorelin, Goserelin, Buserelin, Sermorelin, Nafarelin, Histrelin, Triptorelin, Tesamorelin, Corticorelin, heparin, granulocyte colony-stimulating factor, prostaglandin, ciclosporin, recombinant human interferon $\alpha 2b$, recombinant human interferon b, recombinant human interferon $\gamma$, vasopressin, vancomycin, erythrogenin, Lanreotide Acetate, Terlipressin, Antiangiotide, Nesiritide, Eptifibatide, Sifuvirtide, Atosiban, Ossotide, Muscular Amino Acids and Peptides and Nucleosides, glutathione, mannatide, Enfuvirtide, bradykinin, enkephalin, Nosiheptide, hirudin, Glatiramer acetate, aprotinin, Alarelin, pancreatic kininogenase, polymyxin, Serrapeptase, thymosin $\alpha 1$, thymopentin and thymalfasin.

In one embodiment, the present invention provides a pharmaceutical composition comprising two or more different polypeptides, fish oil, a protease inhibitor, at least one absorption enhancer and at least one surfactant, wherein

the two or more different polypeptides are present in a total mass fraction of 0.1% to 10.0%;

the fish oil is present in a mass fraction of 0.5% to 40.0%;

the protease inhibitor is present in a mass fraction of 5.0% to 28.0%;

the absorption enhancer(s) is/are present in a mass fraction of 8.0% to 42.0%; and

the surfactant(s) is/are present in a mass fraction of 10.0% to 55.0%;

preferably,

the two or more different polypeptides are present in a total mass fraction of 0.9% to 4.6%;

the fish oil is present in a mass fraction of 5.5% to 34.9%;

the protease inhibitor is present in a mass fraction of 10.0% to 22.6%;

the absorption enhancer(s) is/are present in a mass fraction of 12.9% to 36.1%; and

the surfactant(s) is/are present in a mass fraction of 15.0% to 49.6%;

preferably,

the two or more different polypeptides are present in a total mass fraction of 0.9% to 4.6%;

the fish oil is present in a mass fraction of 11.9% to 34.9%;

the protease inhibitor is present in a mass fraction of 10.0% to 22.6%;

the absorption enhancer(s) is/are present in a mass fraction of 12.9% to 36.1%; and

the surfactant(s) is/are present in a mass fraction of 15.0% to 34.5%;

preferably,

the two or more different polypeptides are present in a total mass fraction of 0.9% to 4.6%;

the fish oil is present in a mass fraction of 20.0% to 34.9%;

the protease inhibitor is present in a mass fraction of 10.0% to 17.2%;

the absorption enhancer(s) is/are present in a mass fraction of 12.9% to 35.6%; and

the surfactant(s) is/are present in a mass fraction of 15.0% to 34.5%;

preferably,

the two or more different polypeptides are present in a total mass fraction of 0.9% to 4.6%;

the fish oil is present in a mass fraction of 34.5% to 34.9%;

the protease inhibitor is present in a mass fraction of 10.0% to 17.2%;

the absorption enhancer(s) is/are present in a mass fraction of 12.9% to 35.6%; and

the surfactant(s) is/are present in a mass fraction of 15.0% to 34.5%.

In one embodiment, the present invention provides a pharmaceutical composition, wherein the weight ratio of the fish oil to the surfactant is 1: (0.2 to 15), preferably 1: (0.43 to 9), preferably 1: (0.43 to 5), preferably 1: (0.43 to 3), preferably 1: (0.43 to 2.33), preferably 1: (0.43 to 1), preferably 1:0.5 to 1:2, preferably 1: 0.8 to 1:1.2.

In one embodiment, the present invention provides a pharmaceutical composition, wherein the weight ratio of the fish oil to the surfactant is 1:1, or 1:2.33, or 1:0.43, or 1:9.

In one embodiment of the present invention, the present invention provides use of the oral polypeptide composition, the medicine and the pharmaceutical composition in the preparation of a drug for preventing or treating diabetes, obesity, reduced glucose tolerance, hepatic steatosis, hepatitis, liver fibrosis, cirrhosis and liver cancer, and for improving immunity.

In one embodiment of the present invention, the present invention provides use of the oral polypeptide composition, the medicine and the pharmaceutical composition in the preparation of a drug for preventing or treating a non-alcoholic fatty liver disease, wherein the non-alcoholic fatty liver disease is selected from the group consisting of simple fatty liver and non-alcoholic steatohepatitis.

In one embodiment of the present invention, the present invention provides use of the oral polypeptide composition, the medicine and the pharmaceutical composition in the preparation of a drug for preventing or treating non-alcoholic fatty liver disease complicated with diabetes, non-alcoholic fatty liver disease complicated with nephropathy, non-alcoholic fatty liver disease complicated with cardiovascular disease, non-alcoholic fatty liver disease complicated with obesity, and non-alcoholic fatty liver disease complicated with hyperlipidemia.

In one embodiment of the present invention, the present invention provides a method for treating or preventing a disease, wherein the method comprises orally administering a therapeutically effective amount of the oral polypeptide composition, the medicine or the pharmaceutical composition, to a subject in need thereof, and wherein the disease is selected from the group consisting of diabetes, obesity, reduced glucose tolerance, hepatic steatosis, hepatitis, liver fibrosis, cirrhosis, liver cancer, and immune diseases.

In one embodiment of the present invention, the present invention provides a method for treating or preventing a disease, comprising administering orally to a subject a single dose of 8 to 32 mg of polypeptide in the oral polypeptide composition, 1 to 3 times a day, 1 to 3 capsules per time; or, administering orally to a subject a single dose of 8 to 32 mg of polypeptide in the medicine, 1 to 3 times a day, 1 to 3 capsules per time; or, administering orally to a subject a single dose of 8 to 32 mg of polypeptide in the pharmaceutical composition, 1 to 3 times a day, 1 to 3 capsules per time.

In one embodiment of the present invention, the present invention provides a method for treating or preventing a disease, comprising administering orally to a subject a single dose of 8 mg, 16 mg or 32 mg of polypeptide in the oral polypeptide composition, 1, 2 or 3 times a day, 1, 2 or 3 capsules per time; or, administering orally to a subject a single dose of 8 mg, 16 mg or 32 mg of polypeptide in the medicine, 1, 2 or 3 times a day, 1, 2 or 3 capsules per time; or, administering orally to a subject a single dose of 8 mg, 16 mg or 32 mg of polypeptide in the pharmaceutical compositiont, 1, 2 or 3 times a day, 1, 2 or 3 capsules per time.

In one embodiment of the present invention, the present invention provides an oral polypeptide composition, wherein the oral polypeptide composition comprises a polypeptide, fish oil, and at least one surfactant, and the cumulative dissolution of the polypeptide, when using phosphate buffer solution at pH 6.8 as a dissolution medium, may reach 20% to 95%, or 39% to 95%, or 48% to 95%, or 83% to 95% within 2 minutes.

In one embodiment of the present invention, the oral polypeptide composition is provided, wherein the oral polypeptide composition further comprises a protease inhibitor, and at least one absorption enhancer, and the cumulative dissolution of the polypeptide, when using phosphate buffer solution at pH 6.8 as a dissolution medium, may reach 20% to 95%, or 39% to 95%, or 48% to 95%, or 83% to 95% within 2 minutes.

In one embodiment of the present invention, the present invention provides an oral polypeptide composition, wherein the cumulative dissolution of the polypeptide, when using phosphate buffer solution at pH 6.8 as a dissolution medium, may reach 45% to 101.0%, or 57% to 101.0%, or 93% to 101.0% within5 minutes.

In one embodiment of the present invention, the present invention provides an oral polypeptide composition, wherein the cumulative dissolution of the polypeptide, when using phosphate buffer solution at pH 6.8 as a dissolution medium, may reach 57% to 99.0%, or 73% to 99.0%, or 96.0% to 99.0% within 10 minutes.

In one embodiment of the present invention, the present invention provides an oral polypeptide composition, wherein the cumulative dissolution of the polypeptide when using phosphate buffer solution at pH 6.8 as a dissolution medium, may reach 75% to 99%, or 94% to 99% within 20 minutes.

In one embodiment of the present invention, the present invention provides an oral polypeptide composition, wherein the cumulative dissolution of the polypeptide, when using phosphate buffer solution at pH 6.8 as a dissolution medium, may reach 91% to 100%, or 96% to 100% within 30 minutes.

In one embodiment of the present invention, the present invention provides an oral polypeptide composition, wherein the cumulative dissolution of the polypeptide, when using phosphate buffer solution at pH 6.8 as a dissolution medium, may reach 96% to 100% within 45 minutes.

In one embodiment of the present invention, the present invention provides an oral polypeptide composition, wherein the cumulative dissolution of the polypeptide, when using phosphate buffer solution at pH 6.8 as a dissolution medium, may reach 96% to 100% within 60 minutes.

In one embodiment of the present invention, the present invention provides an oral polypeptide composition, wherein

the polypeptide is present in an amount of 0.1 to 100 parts by weight;
the fish oil is present in an amount of 0.01 to 800 parts by weight;
the protease inhibitor is present in an amount of 0.1 to 400 parts by weight;
the absorption enhancer is present in an amount of 0.1 to 500 parts by weight; and
the surfactant is present in an amount of 1 to 800 parts by weight;
or,
the polypeptide is present in an amount of 0.1 to 100 parts by weight;
the fish oil is present in an amount of 0.01 to 800 parts by weight;
the protease inhibitor is present in an amount of 0.1 to 210 parts by weight;
the absorption enhancer is present in an amount of 0.1 to 300 parts by weight; and
the surfactant is present in an amount of 1 to 800 parts by weight;
or,
the polypeptide is present in an amount of 0.1 to 50 parts by weight;
the fish oil is present in an amount of 0.01 to 600 parts by weight;
the protease inhibitor is present in an amount of 40 to 300 parts by weight;
the absorption enhancer is present in an amount of 80 to 400 parts by weight; and
the surfactant is present in an amount of 50 to 600 parts by weight;
or,
the polypeptide is present in an amount of 5 to 50 parts by weight;
the fish oil is present in an amount of 0.01 to 600 parts by weight;
the protease inhibitor is present in an amount of 40 to 300 parts by weight;
the absorption enhancer is present in an amount of 120 to 400 parts by weight; and
the surfactant is present in an amount of 105 to 450 parts by weight;
or,
the polypeptide is present in an amount of 8 to 32 parts by weight;
the fish oil is present in an amount of 0.01 to 320 parts by weight;
the protease inhibitor is present in an amount of 70 to 200 parts by weight;
the absorption enhancer is present in an amount of 120 to 320 parts by weight; and
the surfactant is present in an amount of 105 to 450 parts by weight;
or,
the polypeptide is present in an amount of 8 to 32 parts by weight;
the fish oil is present in an amount of 50 to 320 parts by weight;
the protease inhibitor is present in an amount of 70 to 200 parts by weight;
the absorption enhancer is present in an amount of 120 to 320 parts by weight; and
the surfactant is present in an amount of 105 to 450 parts by weight;
or,

the polypeptide is present in an amount of 8 to 32 parts by weight;
the fish oil is present in an amount of 105 to 320 parts by weight;
the protease inhibitor is present in an amount of 70 to 200 parts by weight;
the absorption enhancer is present in an amount of 120 to 320 parts by weight; and
the surfactant is present in an amount of 105 to 320 parts by weight.
or,
the polypeptide is present in an amount of 8 to 32 parts by weight;
the fish oil is present in an amount of 245 to 320 parts by weight;
the protease inhibitor is present in an amount of 70 to 160 parts by weight;
the absorption enhancer is present in an amount of 120 to 250 parts by weight; and
the surfactant is present in an amount of 105 to 320 parts by weight.
or,
the polypeptide is present in an amount of 8 to 16 parts by weight;
the fish oil is present in an amount of 105 to 320 parts by weight;
the protease inhibitor is present in an amount of 160 to 200 parts by weight;
the absorption enhancer is present in an amount of 120 to 320 parts by weight; and
the surfactant is present in an amount of 245 to 320 parts by weight.
or,
the polypeptide is present in an amount of 16 to 32 parts by weight;
the fish oil is present in an amount of 105 to 245 parts by weight;
the protease inhibitor is present in an amount of 70 to 200 parts by weight;
the absorption enhancer is present in an amount of 250 to 320 parts by weight; and
the surfactant is present in an amount of 105 to 245 parts by weight.

In one embodiment of the present invention, the present invention provides an oral polypeptide composition, wherein the polypeptide is any one or more selected from the group consisting of insulin or derivatives or analogues thereof, glucagon, glucagon-like peptide-1 receptor agonist, glucagon-like peptide-2 receptor agonist, calcitonin, growth hormone, somatostatin, thyrotropin-releasing hormone, parathyroid hormone, gonadotropin-releasing hormone, heparin, granulocyte colony-stimulating factor, prostaglandin, cyclosporin, interferon, vasopressin, vancomycin, erythrogenin, glutathione and thymosin;

preferably, the polypeptide is any one or more selected from the group consisting of animal insulin, human insulin, recombinant human insulin, insulin derivatives, insulin analogues, glucagon, Exenatide, Benaglutide, Liraglutide, Loxenatide, Dulaglutide, Lixisenatide, Semaglutide, Albiglutide, Dulaglutide, Teduglutide, Lixisenatide, calcitonin, salmon calcitonin, growth hormone, Octreotide, thyrotropin-releasing hormone, parathyroid hormone fragment, Teriparatide, Abaloparatide, Leuprorelin, Protirelin, Gonadorelin, Goserelin, Buserelin, Sermorelin, Nafarelin, Histrelin, Triptorelin, Tesamorelin, Corticorelin, heparin, granulocyte colony-stimulating factor, prostaglandin, cyclosporin, interferon, vasopressin, vancomycin, erythrogenin, Lanreotide Acetate, Terlipressin, Antiangiotide, Nesiritide, Eptifibatide, Sifuvirtide, Atosiban, Ossotide, Muscular Amino Acids and Peptides and Nucleosides, glutathione, mannatide, Enfuvirtide, bradykinin, enkephalin, Nosiheptide, hirudin, Glatiramer acetate, aprotinin, Alarelin, pancreatic kininogenase, polymyxin, Serrapeptase, thymosin $\alpha$1, thymopentin and thymalfasin;
preferably, the polypeptide is any one or more selected from the group consisting of swine insulin, bovine insulin, human insulin, recombinant human insulin, Insulin Lispro, Insulin Aspart, isophane insulin, protamine zinc insulin, Insulin Glargine, Insulin Detemir, Insulin Degludec, glucagon, Exenatide, Benaglutide, Liraglutide, Loxenatide, Dulaglutide, Lixisenatide, Semaglutide, Albiglutide, Dulaglutide, Teduglutide, Lixisenatide, calcitonin, salmon calcitonin, recombinant growth hormone, Octreotide, thyrotropin-releasing hormone, parathyroid hormone fragment, Teriparatide, Abaloparatide, Leuprorelin, Protirelin, Gonadorelin, Goserelin, Buserelin, Sermorelin, Nafarelin, Histrelin, Triptorelin, Tesamorelin, Corticorelin, heparin, granulocyte colony-stimulating factor, prostaglandin, ciclosporin, recombinant human interferon $\alpha$2b, recombinant human interferon b, recombinant human interferon $\gamma$, vasopressin, vancomycin, erythrogenin, Lanreotide Acetate, Terlipressin, Antiangiotide, Nesiritide, Eptifibatide, Sifuvirtide, Atosiban, Ossotide, Muscular Amino Acids and Peptides and Nucleosides, glutathione, mannatide, Enfuvirtide, bradykinin, enkephalin, Nosiheptide, hirudin, Glatiramer acetate, aprotinin, Alarelin, pancreatic kininogenase, polymyxin, Serrapeptase, thymosin $\alpha$1, thymopentin and thymalfasin.

In one embodiment of the present invention, the present invention provides an oral polypeptide composition, wherein the protease inhibitor targets a target protein which is any one or more selected from the group consisting of serine protease, trypsin, chymotrypsin, carboxypeptidase and aminopeptidase;

preferably, the protease inhibitor is any one or more selected from the group consisting of cysteine protease inhibitor, serine protease inhibitor, trypsin inhibitor, threonine protease inhibitor, aspartic protease inhibitor and metalloproteinase inhibitor;

preferably, the protease inhibitor is any one or more selected from the group consisting of soybean trypsin inhibitor, 4-(2-aminoethyl)benzenesulfonylfluoride hydrochloride, ε-aminocaproic acid, antiproteases, α1-antichymotrypsin, antithrombin, α1-antitrypsin, 4-amidinophenyl-methanesulfonyl fluoride, aprotinin, benzamidine-HCl, chymotrypsin, diisopropylfluoro-phosphate, leupeptin, phenylmethylsulfonyl fluoride, 1-chloro-3-sulfonylamino-7-amino-2-heptanone HCl, 1-chloro-3-sulfonylamino-4-phenyl-2-butanone, pentamidine isethionate, egg white trypsin inhibitor, pepstatin, α2-macroglobulin, guanidinium, iodoacetic acid, zinc and chelating agents of zinc.

In one embodiment of the present invention, the present invention provides an oral polypeptide composition, wherein the at least one absorption enhancer is any one or more selected from the group consisting of ethylenediamine tetraacetic acid or salts thereof, N-(8-(2-hydroxybenzoyl)amino)octanoic acid or salts thereof, salicylic acid or salts thereof, citric acid or salts thereof, cholic acid or salts thereof, deoxycholic acid or salts thereof, glycocholic acid or salts thereof, taurocholic acid or salts thereof, alkyl glycosides, sodium dodecyl sulfate, docusate sodium, cyclodextrin or derivatives thereof, chitosan or derivatives thereof, octanoic acid or salts thereof, decanoic acid or salts thereof, lauric acid or salts thereof, myristic acid, palmitic acid, stearic acid, fatty acid triglycerides, lecithins, choline and carnitine.

In one embodiment of the present invention, the present invention provides an oral polypeptide composition, wherein the at least one surfactant is any one or more selected from the group consisting of ionic surfactant, nonionic surfactant and amphoteric surfactant;

preferably, the at least one surfactant is any one or more selected from the group consisting of ammonium dodecyl sulfate, N-dodecyl-β-D-maltoside, tridecyl-β-D-maltoside, sodium dodecyl sulfate, docusate sodium, cyclodextrin or derivatives thereof, chitosan or derivatives thereof, sodium polyoxyethylene alkyl sulfate, sodium laureth sulfate, sodium dioctyl sulfosuccinate, poloxamer, glyceryl monostearate, glycerol monolaurate, sorbitan monolaurate, sorbitan monostearate, polyoxyethylene sorbitan fatty acid esters, sorbitan fatty acid esters, polyoxyethylene monopalmitate, polyoxyethylene hydrogenated castor oil, $C_6$-$C_{12}$ fatty acid triglycerides and $C_8$-$C_{10}$ fatty acid triglycerides;

preferably, the at least one surfactant is selected from the group consisting of Tween, and preferably, the at least one surfactant is any one or more selected from the group consisting of Tween 20, Tween 40, Tween 60, Tween 65, Tween 80 and Tween 85.

In one embodiment of the present invention, the present invention provides an oral polypeptide composition, wherein the weight ratio of the fish oil to the surfactant is 1: (0.2 to 15), preferably 1: (0.43 to 9), preferably 1: (0.43 to 5), preferably 1: (0.43 to 3), preferably 1: (0.43 to 2.33), and preferably 1: (0.43 to 1).

In one embodiment of the present invention, the present invention provides a method for treating or preventing a disease, wherein the method comprises orally administering a therapeutically effective amount of the oral polypeptide composition, the medicine or the pharmaceutical composition, to a subject in need thereof, and wherein the disease is a non-alcoholic fatty liver disease.

In one embodiment of the present invention, the present invention provides a method for treating or preventing a disease, wherein the method comprises orally administering a therapeutically effective amount of the oral polypeptide composition, the medicine or the pharmaceutical composition, to a subject in need thereof, and wherein the disease is selected from the group consisting of non-alcoholic fatty liver disease complicated with diabetes, non-alcoholic fatty liver disease complicated with nephropathy, non-alcoholic fatty liver disease complicated with cardiovascular disease, non-alcoholic fatty liver disease complicated with obesity, and non-alcoholic fatty liver disease complicated with hyperlipidemia.

The present invention provides the following beneficial effects over the prior art:

The present invention provides an oral polypeptide composition that can improve the stability *in vitro* and bioavailability of polypeptide drugs, enabling oral administration.

The present invention provides an oral polypeptide composition that can significantly improve the cumulative dissolution of polypeptide.

## DETAILED DESCRIPTION OF THE INVENTION

Hereinafter, the technical solutions of the present invention will be further explained through specific embodiments in conjunction with the drawings. However, the following examples are only simple examples of the present invention, and do not represent or limit the protective scope of the present invention. The protective scope of the present invention shall be subject to the claims.

In the following examples, unless otherwise specified, all reagents and consumables used were purchased from common reagent manufacturers in this field, and unless otherwise specified, all the experimental methods and technical means used were conventional methods and means in the art.

For example, recombinant human insulin can be purchased from Hefei Tianmai Biotechnology Development Co., Ltd., Omega-3-acid triglycerides meeting the quality standards of European Pharmacopoeia EP10.8 can be used as fish oil, soybean trypsin inhibitor can be purchased from Sigma-Aldrich, disodium ethylenediaminetetraacetate can be purchased from Shanghai Aladdin Biochemical Technology Co., Ltd., and Tween 80 can be purchased from Sigma-Aldrich.

**Example 1**

The present example provides an oral polypeptide composition comprising recombinant human insulin, fish oil, soybean trypsin inhibitor, disodium ethylenediaminetetraacetate and Tween 80 in a mass ratio of 1:40:20:15:40, wherein the mass of recombinant human insulin is 8 mg.

Preparation method: 8 mg of recombinant human insulin, 320 mg of fish oil, 160 mg of soybean trypsin inhibitor, 120 mg of disodium ethylenediaminetetraacetate and 320 mg of Tween 80 were weighed respectively; then, the weighed recombinant human insulin, soybean trypsin inhibitor and disodium ethylenediaminetetraacetate were mixed, and the weighed fish oil and Tween 80 were added thereto, mixed evenly and packed into a gelatin capsule; and the capsule was coated with a coating. The coating materials comprising 40 mg of Eudragit L-100, 40 mg of talcum powder and 5 mg of polyethylene glycol 6000 were dissolved in an appropriate amount of dichloromethane and isopropanol to obtain a coating solution, which was sprayed onto the capsule surface to obtain an oral polypeptide composition.

In the oral polypeptide composition prepared in the present example, the capsule content comprised recombinant human insulin, fish oil, soybean trypsin inhibitor, disodium ethylenediaminetetraacetate, and Tween 80. The capsule content was a suspension and was anhydrous. The term "anhydrous" means that, no water was intentionally added to the capsule content during the process of preparing the oral polypeptide composition of the present example, but it did not exclude the moisture absorbed by the individual components in the capsule content from the air.

Based on the calculation, the oral polypeptide composition obtained in the present example comprised recombinant human insulin present in a mass fraction of 0.9%, fish oil present in a mass fraction of 34.5%, soybean trypsin inhibitor present in a mass fraction of 17.2%, ethylenediamine tetraacetic acid disodium present in a mass fraction of 12.9%, and Tween 80 present in a mass fraction of 34.5%.

**Example 2**

The present example provides an oral polypeptide composition comprising recombinant human insulin, fish oil, soybean trypsin inhibitor, disodium ethylenediaminetetraacetate and Tween 80, wherein the mass of recombinant human insulin is 16 mg.

Preparation method: 16 mg of recombinant human insulin, 105 mg of fish oil, 200 mg of soybean trypsin inhibitor, 320 mg of disodium ethylenediaminetetraacetate and 245 mg of Tween 80 were weighed respectively; then, the weighed recombinant human insulin, soybean trypsin inhibitor and disodium ethylenediaminetetraacetate were mixed, and the weighed fish oil and Tween 80 were added thereto, mixed evenly and packed into a gelatin capsule; and the capsule was coated with a coating. The coating materials comprising 40 mg of Eudragit L-100, 30 mg of talcum powder and 5 mg of polyethylene glycol 6000 were dissolved in an appropriate amount of dichloromethane and isopropanol to obtain a coating solution, which was sprayed onto the capsule surface to obtain an oral polypeptide composition.

In the oral polypeptide composition prepared in the present example, the capsule content comprised recombinant human insulin, fish oil, soybean trypsin inhibitor, disodium ethylenediaminetetraacetate, and Tween 80. The capsule content was a suspension and was anhydrous. The term "anhydrous" means that, no water was intentionally added to the capsule content during the process of preparing the oral polypeptide composition of the present example, but it did not exclude the moisture absorbed by the individual components in the capsule content from the air.

Based on the calculation, the oral polypeptide composition obtained in the present example comprised recombinant human insulin present in a mass fraction of 1.8 %, fish oil present in a mass fraction of 11.9%, soybean trypsin inhibitor present in a mass fraction of 22.6 %, disodium ethylenediaminetetraacetate present in a mass fraction of 36.1 %, and Tween 80 present in a mass fraction of 27.7%.

Example 3

The present example provides an oral polypeptide composition comprising recombinant human insulin, fish oil, soybean trypsin inhibitor, disodium ethylenediaminetetraacetate and Tween 80, wherein the mass of recombinant human insulin is 32 mg.

Preparation method: 32 mg of recombinant human insulin, 245 mg of fish oil, 70 mg of soybean trypsin inhibitor, 250 mg of disodium ethylenediaminetetraacetate and 105 mg of Tween 80 were weighed respectively; then, the weighed recombinant human insulin, soybean trypsin inhibitor and disodium ethylenediaminetetraacetate were mixed, and the weighed fish oil and Tween 80 were added thereto, mixed evenly and packed into a gelatin capsule; and the capsule was coated with a coating. The coating materials comprising 40 mg of Eudragit L-100, 30 mg of talcum powder and 5 mg of polyethylene glycol 6000 were dissolved in an appropriate amount of dichloromethane and isopropanol to obtain a coating solution, which was sprayed onto the capsule surface to obtain an oral polypeptide composition.

In the oral polypeptide composition prepared in the present example, the capsule content comprised recombinant human insulin, fish oil, soybean trypsin inhibitor, disodium ethylenediaminetetraacetate and Tween 80. The capsule content was a suspension and was anhydrous. The term "anhydrous" means that no water was intentionally added to the capsule conten, during the process of preparing the oral polypeptide composition of the present example, but it did not exclude the moisture absorbed by the individual components in the capsule content from the air.

Based on the calculation, the oral polypeptide composition obtained in the present example comprised recombinant human insulin present in a mass fraction of 4.6%, fish oil present in a mass fraction of 34.9%, soybean trypsin inhibitor present in a mass fraction of 10.0%, disodium ethylenediaminetetraacetate present in a mass fraction of 35.6%, and Tween 80 present in a mass fraction of 15.0%.

**Example 4**

The present example provides an oral polypeptide composition comprising recombinant human insulin, fish oil, soybean trypsin inhibitor, disodium ethylenediaminetetraacetate and Tween 80, wherein the mass of recombinant human insulin is 8 mg.

Preparation method: 8 mg of recombinant human insulin, 50 mg of fish oil, 100 mg of soybean trypsin inhibitor, 300 mg of disodium ethylenediaminetetraacetate and 450 mg of Tween 80 were weighed respectively; then, the weighed recombinant human insulin, soybean trypsin inhibitor and disodium ethylenediaminetetraacetate were mixed, and the weighed fish oil and Tween 80 were added thereto, mixed evenly and packed into a gelatin capsule; and the capsule was coated with a coating. The coating materials comprising 40 mg of Eudragit L-100, 30 mg of talcum powder and 5 mg of polyethylene glycol 6000 were dissolved in an appropriate amount of dichloromethane and isopropanol to obtain a coating solution, which was sprayed onto the capsule surface to obtain an oral polypeptide composition.

In the oral polypeptide composition prepared in the present example, the capsule content comprised recombinant human insulin, fish oil, soybean trypsin inhibitor, disodium ethylenediaminetetraacetate, and Tween 80. The capsule content was a suspension and was anhydrous. The term "anhydrous" means that no water was intentionally added to the capsule content during the process of preparing the oral polypeptide composition of the present example, but it did not exclude the moisture absorbed by individual components in the capsule content from the air.

Based on the calculation, the oral polypeptide composition obtained in the present example comprised recombinant human insulin present in a mass fraction of 0.9%, fish oil present in a mass fraction of 5.5%, soybean trypsin inhibitor present in a mass fraction of 11.0%, disodium ethylenediaminetetraacetate present in a mass fraction of 33.0%, and Tween 80 present in a mass fraction of 49.6%.

**Example 5-Comparative Example**

The present example provides an oral polypeptide composition comprising recombinant human insulin, soybean trypsin inhibitor, disodium ethylenediaminetetraacetate and Tween 80, wherein the mass of recombinant human insulin is 16 mg.

Preparation method: 16 mg of recombinant human insulin, 200 mg of soybean trypsin inhibitor, 320 mg of disodium ethylenediaminetetraacetate and 350 mg of Tween 80 were weighed respectively; then, the weighed recombinant human insulin, soybean trypsin inhibitor and disodium ethylenediaminetetraacetate were mixed, and the weighed Tween 80 was added thereto, mixed evenly and packed into a gelatin capsule; and the capsule was coated with a coating. The coating materials comprising 40 mg of Eudragit L-100, 30 mg of talcum powder and 5 mg of polyethylene glycol 6000 were dissolved in an appropriate amount of dichloromethane and isopropanol to obtain a coating solution, which was sprayed onto the capsule surface to obtain an oral polypeptide composition.

**Example 6-Comparative Example**

The present example provides an oral polypeptide composition comprising recombinant human insulin, fish oil, soybean trypsin inhibitor and disodium ethylenediaminetetraacetate, wherein the mass of recombinant human insulin is 16 mg.

Preparation method: 16 mg of recombinant human insulin, 350 mg of fish oil, 200 mg of soybean trypsin inhibitor and 320 mg of disodium ethylenediaminetetraacetate were weighed respectively; then, the weighed recombinant human insulin, soybean trypsin inhibitor and disodium ethylenediaminetetraacetate were mixed, and the weighed fish oil was added thereto, mixed evenly and packed into a gelatin capsule; and the capsule was coated with a coating. The coating materials comprising 40 mg of Eudragit L-100, 30 mg of talcum powder and 5 mg of polyethylene glycol 6000 were dissolved in an appropriate amount of dichloromethane and isopropanol to obtain a coating solution, which was sprayed onto the capsule surface to obtain an oral polypeptide composition.

**Example 7**

Stability test

HPLC was used to detect the content of recombinant human insulin in the oral polypeptide compositions of Examples 1, 2, 3 and 4 after placing at 25°C for 0, 5, 10, 30, and 60 days, and three samples were detected for each oral polypeptide composition at each time point. The obtained results are shown in Table 1, in which the content is the average value of the three samples at each time point.

Table 1: Stability data of recombinant human insulin at 25°C

| Oral polypeptide composition | Content of recombinant human insulin (%) | | | | |
|---|---|---|---|---|---|
| | Day 0 | Day 5 | Day 10 | Day 30 | Day 60 |
| Example 1 | 100.9% | 99.0% | 96.3% | 86.1% | 63.3% |
| Example 2 | 99.8% | 99.3% | 97.5% | 88.7% | 68.1% |
| Example 3 | 101.2% | 100.4% | 95.8% | 83.5% | 62.1% |
| Example 4 | 100.5% | 99.6% | 98.1% | 89.2% | 69.4% |

"Content of recombinant human insulin" refers to the percentage of the actual content of recombinant human insulin relative to the labeled amount. For example, if recombinant human insulin in an oral polypeptide composition has a labeled amount of 8 mg, and after being left for a period of time, three samples are taken for HPLC detection and the actual recombinant human insulin (average value) is calculated as 4.8 mg, the content of recombinant human insulin in the oral polypeptide composition is $\frac{4.8\ mg}{8.0\ mg}\times100\%=60\%$.

HPLC conditions for determining the content of recombinant human insulin:

Chromatography column: octadecylsilane bonded silica gel;
Mobile phase A: 600 mL of 0.1 M sodium sulfate buffer (pH 4.8), 100 mL of acetonitrile, and 300 mL of water;
Mobile phase B: 100 mL of 0.1 M sodium sulfate buffer (pH 4.8), 600 mL of acetonitrile, and 300 mL of water;
Flow rate: 1.0 ml/min; Column temperature: 35°C; Detection wavelength: UV 214 nm; Injection volume: 20 µl.

The gradient elution is listed as follows:

Table 1.1: Gradient elution list

| Time (min) | Mobile phase A (%) | Mobile phase B (%) |
|---|---|---|
| 0 | 80 | 20 |
| 15 | 80 | 20 |
| 16 | 0 | 100 |
| 21 | 0 | 100 |
| 22 | 90 | 10 |
| 30 | 90 | 10 |

According to Table 1, the recombinant human insulin content in the oral peptide compositions was basically unchanged after placing at 25°C for 5 days, greater than 95% after placing for 10 days, greater than 83% after placing for 30 days, and greater than 62% after placing for 60 days.

HPLC was used to detect the content of recombinant human insulin in the oral polypeptide compositions of Examples 1, 2, 3 and 4 after placing at 40°C for 0, 5, and 10 days, and three samples were detected for each oral polypeptide composition at each time point. The obtained results are shown in Table 2, in which the content is the average value of the three samples at each time point.

Table 2: Stability data of recombinant human insulin at 40°C

| Oral polypeptide composition | Content of recombinant human insulin (%) | | |
|---|---|---|---|
| | Day 0 | Day 5 | Day 10 |
| Example 1 | 100.9% | 86.9% | 79.1% |
| Example 2 | 99.8% | 87.1% | 81.2% |
| Example 3 | 101.2% | 84.3% | 77.7% |
| Example 4 | 100.5% | 88.2% | 80.9% |

According to Table 2, the recombinant human insulin content in the oral peptide compositions was greater than 84% after placing at 40°C for 5 days, and greater than 75% after placing for 10 days.

**Example 8**

Dissolution test

According to the dissolution determination method specified in the second method of General Rule 0931 in Volume IV of the Chinese Pharmacopoeia 2020 edition, the dissolution test was performed as follows: phosphate buffer solution at pH 6.8 was used as a dissolution medium, the contents were taken from the oral polypeptide compositions prepared in Examples 1, 2, 3, 4, 5 and 6, poured into different dissolution cups and dissolved in the above dissolution medium, rotated at 50 revolutions per minute, and the dissolution medium was kept at a constant temperature of 37°C $\pm$ 0.5°C. Samples were taken at 0 min, 2 min, 5 min, 10 min, 20 min, 30 min, 45 min, and 60 min, and the content of recombinant human insulin was measured by HPLC. The cumulative dissolution data are shown in Table 3.

Table 3: Cumulative dissolution data of recombinant human insulin

| Time Cumulative Dissolution Example | 0 min | 2 min | 5 min | 10 min | 20 min | 30 min | 45 min | 60 min |
|---|---|---|---|---|---|---|---|---|
| Example 5 | 0% | 19.94% | 27.12% | 36.47% | 57.40% | 73.76% | 91.62% | 96.95% |
| Example 4 | 0% | 38.56% | 44.85% | 56.57% | 75.23% | 91.56% | 99.15% | 96.13% |
| Example 2 | 0% | 48.03% | 57.23% | 73.16% | 94.46% | 98.25% | 97.66% | 98.15% |
| Example 1 | 0% | 82.54% | 93.26% | 99.14% | 98.76% | 100.65% | 97.61% | 100.03% |
| Example 3 | 0% | 94.83% | 101.28% | 96.13% | 99.09% | 96.35% | 99.07% | 96.58% |
| Example 6 | 0% | 7.07% | 10.81% | 14.37% | 19.88% | 24.78% | 32.95% | 42.17% |

HPLC conditions for determining the content of recombinant human insulin:

Chromatography column: octadecylsilane bonded silica gel;

Mobile phase A: 600 mL of 0.1 M sodium sulfate buffer (pH 4.8), 100 mL of acetonitrile, and 300 mL of water;
Mobile phase B: 100 mL of 0.1 M sodium sulfate buffer (pH 4.8), 600 mL of acetonitrile, and 300 mL of water;
Flow rate: 1.0 ml/min; Column temperature: 35°C; Detection wavelength: UV 214 nm; Injection volume is 20 µl.

The gradient elution is listed as follows:

Table 3.1: Gradient elution list

| Time (min) | Mobile phase A (%) | Mobile phase B (%) |
| --- | --- | --- |
| 0 | 80 | 20 |
| 15 | 80 | 20 |
| 16 | 0 | 100 |
| 21 | 0 | 100 |
| 22 | 90 | 10 |
| 30 | 90 | 10 |

It can be seen from Table 3 that,

the oral polypeptide composition of Example 4 has a cumulative dissolution of greater than 90% within 30 minutes and almost complete dissolution within 45 minutes;
the oral polypeptide composition of Example 2 has a cumulative dissolution of greater than 90% within 20 minutes and almost complete dissolution within 30 minutes;
the oral polypeptide compositions of Examples 1 and 3 have a cumulative dissolution of greater than 80% within 2 minutes, greater than 90% within 5 minutes, and almost complete dissolution within 10 minutes.

Dissolution test

According to the dissolution determination method specified in the second method of General Rule 0931 in Volume IV of the Chinese Pharmacopoeia 2020 edition, the dissolution test was performed as follows: phosphate buffer solution at pH 6.8 was used as a dissolution medium, the contents were taken from the oral polypeptide compositions prepared in Examples 1, 2, 3 and 4, poured into different dissolution cups and dissolved in the above dissolution medium, rotated at 50 revolutions per minute, and the dissolution medium was kept at a constant temperature of 37°C ± 0.5°C. Samples were taken at 0 min, 2 min, 5 min, 10 min, 20 min, 30 min, 45 min, and 60 min, and the content of soybean trypsin inhibitor was measured by HPLC. The cumulative dissolution data are shown in Table 4.

Table 4: Cumulative dissolution data of soybean trypsin inhibitor

| Time / Cumulative dissolution (%) | 0 min | 2 min | 5 min | 10 min | 20 min | 30 min | 45 min | 60 min |
| --- | --- | --- | --- | --- | --- | --- | --- | --- |
| | | | | | | | | |

| Example | | | | | | | | |
|---|---|---|---|---|---|---|---|---|
| Example 4 | 0 | 46.24 | 58.05 | 69.03 | 79.68 | 84.80 | 89.36 | 89.06 |
| Example 2 | 0 | 69.90 | 79.56 | 87.13 | 90.52 | 89.80 | 89.24 | 89.95 |
| Example 1 | 0 | 86.60 | 91.73 | 92.71 | 89.09 | 89.83 | 92.27 | 92.98 |
| Example 3 | 0 | 90.72 | 91.13 | 90.81 | 89.86 | 91.82 | 92.75 | 91.35 |

HPLC conditions for determining the content of soybean trypsin inhibitor:

Mobile phase A: Water-acetonitrile-trifluoroacetic acid (95 ml: 5 ml: 0.05 ml);
Mobile phase B: Water-acetonitrile-trifluoroacetic acid (10 ml: 90 ml: 0.05 ml);
Chromatographic column: C18;
Flow rate: 1 mL/min;
Column temperature: 40°C;
Detector: UV 214 nm;
Injection volume: 20 μL;

The gradient elution is listed as follows:

Table 4.1: Gradient elution list

| N | Time (min) | Mobile phase A (%) | Mobile phase B (%) |
|---|---|---|---|
| 1 | 0 | 100 | 0 |
| 2 | 15 | 40 | 60 |
| 4 | 15.01 | 100 | 0 |
| 5 | 25 | 100 | 0 |
| 8 | 25.01 | 100 | 0 |
| 9 | 35 | 100 | 0 |

**Example 9**

Animal experiment

The animal experiment was conducted with the oral polypeptide compositions prepared in Examples 1-4.

(1) Experimental animals

Beagle dogs, male, 8-10 kg.

(2) Animal grouping and administration design

Group design: four groups were divided for the experiment: low-dose group 1, medium-dose group, high-dose group, and low-dose group 2;
Number of animals: 8 male dogs in each administration group;
Experimental method: two rounds of experiments were carried out, each consisting of 4 groups with 4 animals per group. A washout period was set to 48 hours;
Grouping method: animals were randomly grouped depending on body weight. The specific grouping information is shown in Table 5.

Table 5: Dose and grouping information list

| Group | | Solvent/t est substanc e | Dose administ ered | Quantity administ ered | Route of administra tion | Frequency of administra tion | Num ber of anim als | Anima l numbe r[1] |
|---|---|---|---|---|---|---|---|---|
| | | The first round of experiment | | | | | | |
| 1 | Low-dose group 1 | Oral polypept ide composi tion of Example 1 | 8 mg/anim al | One capsule | Oral administra tion | Once | 4 | 1M00 1-1M00 4 |
| 2 | Mediu m-dose group | Oral polypept ide composi tion of Example 2 | 16 mg/anim al | One capsule | Oral administra tion | Once | 4 | 2M00 1-2M00 4 |
| 3 | High-dose group | Oral polypept ide composi tion of Example 3 | 32 mg/anim al | One capsule | Oral administra tion | Once | 4 | 3M00 1-3M00 4 |
| 4 | Low-dose group 2 | Oral polypept ide composi tion of Example 4 | 8 mg/anim al | One capsule | Oral administra tion | Once | 4 | 4M00 1-4M00 4 |
| The second round of experiment | | | | | | | | |
| 1 | Low-dose group 1 | Oral polypept ide composi tion of Example 1 | 8 mg/anim al | One capsule | Oral administra tion | Once | 4 | 1M00 5-1M00 8 |
| 2 | Mediu m-dose group | Oral polypept ide composi tion of | 16 mg/anim al | One capsule | Oral administra tion | Once | 4 | 2M00 5-2M00 8 |
| Group | | Solvent/t est substanc e | Dose administ ered | Quantity administ ered | Route of administra tion | Frequency of administra tion | Num ber of anim als | Anima l numbe r[1] |
| | | Example 2 | | | | | | |
| 3 | High-dose group | Oral polypept ide composi tion of Example 3 | 32 mg/anim al | One capsule | Oral administra tion | Once | 4 | 3M00 5-3M00 8 |
| 4 | Low-dose group 2 | Oral polypept ide composi tion of Example 4 | 8 mg/anim al | One capsule | Oral administra tion | Once | 4 | 4M00 5-4M00 8 |
| Remark: [1] The first digit of the animal number represents the group, the second letter represents gender (M for male), and the digits in positions 3, 4 and 5 represent the individual number of the animal; [2] After the washout period of 48 hours after the end of the first round of experiment, the groups were randomly divided for the second round; [3] Fasting overnight for at least 12 hours before administration. | | | | | | | | |

(3) Administration method

Low-dose group 1, medium-dose group, high-dose group, and low-dose group 2
Route of administration: oral administration;
Frequency of administration: administrated once;
Quantity administered: one capsule;

(4) Blood glucose testing

Blood was collected from forelimb veins before and after administration, and the blood glucose was detected using a blood glucose meter.

Conclusion:

The animals in the low-dose group 1 began to show a decrease in blood glucose at 0.667 h, with the blood glucose returning to normal by 2.5 h. The maximum hypoglycemic effect was reached at 1.33 h, with a maximum blood glucose reduction rate of 49.72%;

The animals in the medium-dose group began to show a decrease in blood glucose at 1.00 h, with the blood glucose returning to normal by 2.75 h. The maximum hypoglycemic effect was reached at 1.33 h, with a maximum blood glucose reduction rate of 53.41%;

The animals in the high-dose group begna to show a decrease in blood glucose at 0.667 h, with the blood glucose returning to normal by 3.5 h. The maximum hypoglycemic effect was reached at 1.75 h, with a maximum blood glucose reduction rate of 61.18%;

The animals in the low-dose group 2 began to show a decrease in blood glucose at 0.667 h, with the blood glucose returning to normal by 2.5 h. The maximum hypoglycemic effect was reached at 1.33 h, with a maximum blood glucose reduction rate of 50.23%.

The blood glucose reduction rate was calculated by: (average blood glucose value at each time point - blood glucose value at 0 h) / blood glucose value at 0 h.

The applicant declares that the examples above are only the preferable examples of the present invention, and merely intended to describe the technical solutions of the present invention, but not to limit the protective scope of the present invention. Any modification, equivalent replacement or improvement made according to the spirit and principle of the present invention shall be included within the protective scope of the present invention.

**Claims**

1. An oral polypeptide composition comprising a polypeptide, fish oil, a protease inhibitor, at least one absorption enhancer and at least one surfactant, wherein,

   the polypeptide is present in an amount of 0.1 to 100 parts by weight;
   the fish oil is present in an amount of 0.01 to 800 parts by weight;
   the protease inhibitor is present in an amount of 0.1 to 210 parts by weight; the absorption enhancer(s) is/are present in an amount of 0.1 to 320 parts by weight; and
   the surfactant(s) is/are present in an amount of 1 to 800 parts by weight;

   preferably,

   the polypeptide is present in an amount of 5 to 50 parts by weight;
   the fish oil is present in an amount of 30 to 400 parts by weight;
   the protease inhibitor is present in an amount of 40 to 210 parts by weight;
   the absorption enhancer(s) is/are present in an amount of 60 to 320 parts by weight; and
   the surfactant(s) is/are present in an amount of 50 to 550 parts by weight;

   preferably,

   the polypeptide is present in an amount of 8 to 32 parts by weight;
   the fish oil is present in an amount of 50 to 320 parts by weight;
   the protease inhibitor is present in an amount of 70 to 200 parts by weight;
   the absorption enhancer(s) is/are present in an amount of 120 to 320 parts by weight; and
   the surfactant(s) is/are present in an amount of 105 to 450 parts by weight;

   preferably,

   the polypeptide is present in an amount of 8 to 32 parts by weight;
   the fish oil is present in an amount of 105 to 320 parts by weight;

the protease inhibitor is present in an amount of 70 to 200 parts by weight;
the absorption enhancer(s) is/are present in an amount of 120 to 320 parts by weight; and
the surfactant(s) is/are present in an amount of 105 to 320 parts by weight;

preferably,

the polypeptide is present in an amount of 8 to 32 parts by weight;
the fish oil is present in an amount of 245 to 320 parts by weight;
the protease inhibitor is present in an amount of 70 to 160 parts by weight;
the absorption enhancer(s) is/are present in an amount of 120 to 250 parts by weight; and
the surfactant(s) is/are present in an amount of 105 to 320 parts by weight.

2. The oral polypeptide composition according to claim 1, wherein the oral polypeptide composition comprises a polypeptide, fish oil, a protease inhibitor, at least one absorption enhancer and at least one surfactant, wherein

the polypeptide is present in an amount of 1 part by weight;
the fish oil is present in an amount of 0.01 to 800 parts by weight;
the protease inhibitor is present in an amount of 0.1 to 210 parts by weight;
the absorption enhancer(s) is/are present in an amount of 0.1 to 300 parts by weight; and
the surfactant(s) is/are present in an amount of 1 to 800 parts by weight;

preferably,

the polypeptide is present in an amount of 1 part by weight;
the fish oil is present in an amount of 3 to 50 parts by weight;
the protease inhibitor is present in an amount of 1 to 30 parts by weight;
the absorption enhancer(s) is/are present in an amount of 5 to 50 parts by weight; and
the surfactant(s) is/are present in an amount of 3 to 65 parts by weight;

preferably,

the polypeptide is present in an amount of 1 part by weight;
the fish oil is present in an amount of 6.3 to 40 parts by weight;
the protease inhibitor is present in an amount of 2.2 to 20 parts by weight;
the absorption enhancer(s) is/are present in an amount of 7.8 to 37.5 parts by weight; and
the surfactant(s) is/are present in an amount of 3.3 to 56.3 parts by weight;

preferably,

the polypeptide is present in an amount of 1 part by weight;
the fish oil is present in an amount of 6.6 to 40 parts by weight;
the protease inhibitor is present in an amount of 2.2 to 20 parts by weight;
the absorption enhancer(s) is/are present in an amount of 7.8 to 20 parts by weight; and
the surfactant(s) is/are present in an amount of 3.3 to 40 parts by weight;

preferably,

the polypeptide is present in an amount of 1 part by weight;
the fish oil is present in an amount of 7.7 to 40 parts by weight;
the protease inhibitor is present in an amount of 2.2 to 20 parts by weight;
the absorption enhancer(s) is/are present in an amount of 7.8 to 15 parts by weight; and
the surfactant(s) is/are present in an amount of 3.3 to 40 parts by weight.

3. The oral polypeptide composition according to claim 1, wherein the oral polypeptide composition comprises a polypeptide, fish oil, a protease inhibitor, at least one absorption enhancer and at least one surfactant, wherein

the polypeptide is present in a mass fraction of 0.1% to 10.0%;
the fish oil is present in a mass fraction of 0.5% to 40.0%;

the protease inhibitor is present in a mass fraction of 5.0% to 28.0%;
the absorption enhancer(s) is/are present in a mass fraction of 8.0% to 42.0%; and
the surfactant(s) is/are present in a mass fraction of 10.0% to 55.0%;

preferably,

the polypeptide is present in a mass fraction of 0.9% to 4.6%;
the fish oil is present in a mass fraction of 5.5% to 34.9%;
the protease inhibitor is present in a mass fraction of 10.0% to 22.6%;
the absorption enhancer(s) is/are present in a mass fraction of 12.9% to 36.1%; and
the surfactant(s) is/are present in a mass fraction of 15.0% to 49.6%;

preferably,

the polypeptide is present in a mass fraction of 0.9% to 4.6%;
the fish oil is present in a mass fraction of 11.9% to 34.9%;
the protease inhibitor is present in a mass fraction of 10.0% to 22.6%;
the absorption enhancer(s) is/are present in a mass fraction of 12.9% to 36.1%; and
the surfactant(s) is/are present in a mass fraction of 15.0% to 34.5%;

preferably,

the polypeptide is present in a mass fraction of 0.9% to 4.6%;
the fish oil is present in a mass fraction of 20.0% to 34.9%;
the protease inhibitor is present in a mass fraction of 10.0% to 17.2%;
the absorption enhancer(s) is/are present in a mass fraction of 12.9% to 35.6%; and
the surfactant(s) is/are present in a mass fraction of 15.0% to 34.5%;

preferably,

the polypeptide is present in a mass fraction of 0.9% to 4.6%;
the fish oil is present in a mass fraction of 34.5% to 34.9%;
the protease inhibitor is present in a mass fraction of 10.0% to 17.2%;
the absorption enhancer(s) is/are present in a mass fraction of 12.9% to 35.6%; and
the surfactant(s) is/are present in a mass fraction of 15.0% to 34.5%;

preferably,

the polypeptide is present in a mass fraction of 0.9% to 1.8%;
the fish oil is present in a mass fraction of 11.9% to 34.5%;
the protease inhibitor is present in a mass fraction of 17.2% to 22.6%;
the absorption enhancer(s) is/are present in a mass fraction of 12.9% to 36.1%; and
the surfactant(s) is/are present in a mass fraction of 27.7% to 34.5%;

preferably,

the polypeptide is present in a mass fraction of 1.8% to 4.6%;
the fish oil is present in a mass fraction of 11.9% to 34.9%;
the protease inhibitor is present in a mass fraction of 10.0% to 22.6%;
the absorption enhancer(s) is/are present in a mass fraction of 35.6% to 36.1%; and
the surfactant(s) is/are present in a mass fraction of 15.0% to 27.7%.

4. The oral polypeptide composition according to claim 1, wherein the oral polypeptide composition comprises a polypeptide, fish oil, a protease inhibitor, at least one absorption enhancer and at least one surfactant; and wherein in unit dosage form,

the polypeptide is present in an amount of 8 to 32 mg;
the fish oil is present in an amount of 50 to 320 mg;

the protease inhibitor is present in an amount of 70 to 200 mg;
the absorption enhancer(s) is/are present in an amount of 120 to 320 mg; and
the surfactant(s) is/are present in an amount of 105 to 450 mg;

preferably,

the polypeptide is present in an amount of 8 to 32 mg;
the fish oil is present in an amount of 105 to 320 mg;
the protease inhibitor is present in an amount of 70 to 200 mg;
the absorption enhancer(s) is/are present in an amount of 120 to 320 mg; and
the surfactant(s) is/are present in an amount of 105 to 320 mg;

preferably,

the polypeptide is present in an amount of 8 to 32 mg;
the fish oil is present in an amount of 245 to 320 mg;
the protease inhibitor is present in an amount of 70 to 160 mg;
the absorption enhancer(s) is/are present in an amount of 120 to 250 mg; and
the surfactant(s) is/are present in an amount of 105 to 320 mg.

5. The oral polypeptide composition according to claim 1, wherein the oral polypeptide composition comprises a polypeptide, fish oil, a protease inhibitor, at least one absorption enhancer and at least one surfactant; and wherein in unit dosage form,

the polypeptide is present in an amount of 8 mg;
the fish oil is present in an amount of 320 mg;
the protease inhibitor is present in an amount of 160 mg;
the absorption enhancer(s) is/are present in an amount of 120 mg; and
the surfactant(s) is/are present in an amount of 320 mg;
or,
the polypeptide is present in an amount of 16 mg;
the fish oil is present in an amount of 105 mg;
the protease inhibitor is present in an amount of 200 mg;
the absorption enhancer(s) is/are present in an amount of 320 mg; and
the surfactant(s) is/are present in an amount of 245 mg;
or,
the polypeptide is present in an amount of 32 mg;
the fish oil is present in an amount of 245 mg;
the protease inhibitor is present in an amount of 70 mg;
the absorption enhancer(s) is/are present in an amount of 250 mg; and
the surfactant(s) is/are present in an amount of 105 mg;
or,
the polypeptide is present in an amount of 8 mg;
the fish oil is present in an amount of 50 mg;
the protease inhibitor is present in an amount of 100 mg;
the absorption enhancer(s) is/are present in an amount of 300 mg; and
the surfactant(s) is/are present in an amount of 450 mg.

6. The oral polypeptide composition according to claim 1, wherein the polypeptide has a molecular weight of 100 Da to 20000 Da, preferably 1000 Da to 20000 Da, more preferably 1000 Da to 6000 Da, and further preferably 2000 Da to 6000 Da.

7. The oral polypeptide composition according to claim 1, wherein the polypeptide is any one or more selected from the group consisting of insulin or derivatives or analogues thereof, glucagon, glucagon-like peptide-1 receptor agonist, glucagon-like peptide-2 receptor agonist, calcitonin, growth hormone, somatostatin, thyrotropin-releasing hormone, parathyroid hormone, gonadotropin-releasing hormone, heparin, granulocyte colony-stimulating factor, prostaglandin, cyclosporin, interferon, vasopressin, vancomycin, erythrogenin, glutathione and thymosin;

preferably, the polypeptide is any one or more selected from the group consisting of animal insulin, human insulin, recombinant human insulin, insulin derivatives, insulin analogues, glucagon, Exenatide, Benaglutide, Liraglutide, Loxenatide, Dulaglutide, Lixisenatide, Semaglutide, Albiglutide, Dulaglutide, Teduglutide, Lixisenatide, calcitonin, salmon calcitonin, growth hormone, Octreotide, thyrotropin-releasing hormone, parathyroid hormone fragment, Teriparatide, Abaloparatide, Leuprorelin, Protirelin, Gonadorelin, Goserelin, Buserelin, Sermorelin, Nafarelin, Histrelin, Triptorelin, Tesamorelin, Corticorelin, heparin, granulocyte colony-stimulating factor, prostaglandin, cyclosporin, interferon, vasopressin, vancomycin, erythrogenin, Lanreotide Acetate, Terlipressin, Antiangiotide, Nesiritide, Eptifibatide, Sifuvirtide, Atosiban, Ossotide, Muscular Amino Acids and Peptides and Nucleosides, glutathione, mannatide, Enfuvirtide, bradykinin, enkephalin, Nosiheptide, hirudin, Glatiramer acetate, aprotinin, Alarelin, pancreatic kininogenase, polymyxin, Serrapeptase, thymosin $\alpha1$, thymopentin and thymalfasin;

preferably, the polypeptide is any one or more selected from the group consisting of swine insulin, bovine insulin, human insulin, recombinant human insulin, Insulin Lispro, Insulin Aspart, isophane insulin, protamine zinc insulin, Insulin Glargine, Insulin Detemir, Insulin Degludec, glucagon, Exenatide, Benaglutide, Liraglutide, Loxenatide, Dulaglutide, Lixisenatide, Semaglutide, Albiglutide, Dulaglutide, Teduglutide, Lixisenatide, calcitonin, salmon calcitonin, recombinant growth hormone, Octreotide, thyrotropin-releasing hormone, parathyroid hormone fragment, Teriparatide, Abaloparatide, Leuprorelin, Protirelin, Gonadorelin, Goserelin, Buserelin, Sermorelin, Nafarelin, Histrelin, Triptorelin, Tesamorelin, Corticorelin, heparin, granulocyte colony-stimulating factor, prostaglandin, ciclosporin, recombinant human interferon $\alpha2b$, recombinant human interferon b, recombinant human interferon $\gamma$, vasopressin, vancomycin, erythrogenin, Lanreotide Acetate, Terlipressin, Antiangiotide, Nesiritide, Eptifibatide, Sifuvirtide, Atosiban, Ossotide, Muscular Amino Acids and Peptides and Nucleosides, glutathione, mannatide, Enfuvirtide, bradykinin, enkephalin, Nosiheptide, hirudin, Glatiramer acetate, aprotinin, Alarelin, pancreatic kininogenase, polymyxin, Serrapeptase, thymosin $\alpha1$, thymopentin and thymalfasin.

8. The oral polypeptide composition according to claim 1, wherein the protease inhibitor targets a target protein which is any one or more selected from the group consisting of serine protease, trypsin, chymotrypsin, carboxypeptidase and aminopeptidase;

preferably, the protease inhibitor is any one or more selected from the group consisting of cysteine protease inhibitor, serine protease inhibitor, trypsin inhibitor, threonine protease inhibitor, aspartic protease inhibitor and metalloproteinase inhibitor;

preferably, the protease inhibitor is any one or more selected from the group consisting of soybean trypsin inhibitor, 4-(2-aminoethyl)benzenesulfonylfluoride hydrochloride, $\epsilon$-aminocaproic acid, antiproteases, $\alpha1$-antichymotrypsin, antithrombin, $\alpha1$-antitrypsin, 4-amidinophenyl-methanesulfonyl fluoride, aprotinin, benzamidine-HCl, chymotrypsin, diisopropylfluoro-phosphate, leupeptin, phenylmethylsulfonyl fluoride, 1-chloro-3-sulfonylamino-7-amino-2-heptanone HCl, 1-chloro-3-sulfonylamino-4-phenyl-2-butanone, pentamidine isethionate, egg white trypsin inhibitor, pepstatin, $\alpha2$-macroglobulin, guanidinium, iodoacetic acid, zinc and chelating agents of zinc.

9. The oral polypeptide composition according to claim 1, wherein the at least one absorption enhancer is any one or more selected from the group consisting of ethylenediamine tetraacetic acid or salts thereof, N-(8-(2-hydroxybenzoyl) amino)octanoic acid or salts thereof, salicylic acid or salts thereof, citric acid or salts thereof, cholic acid or salts thereof, deoxycholic acid or salts thereof, glycocholic acid or salts thereof, taurocholic acid or salts thereof, alkyl glycosides, sodium dodecyl sulfate, docusate sodium, cyclodextrin or derivatives thereof, chitosan or derivatives thereof, octanoic acid or salts thereof, decanoic acid or salts thereof, lauric acid or salts thereof, myristic acid, palmitic acid, stearic acid, fatty acid triglycerides, lecithins, choline and carnitine, and preferably, the salt of ethylenediamine tetraacetic acid is selected from the group consisting of disodium ethylenediaminetetraacetate, dipotassium ethylenediaminetetraacetate, trisodium ethylenediaminetetraacetate or tetrasodium ethylenediaminetetraacetate.

10. The oral polypeptide composition according to claim 1, wherein the at least one surfactant is any one or more selected from the group consisting of ionic surfactant, nonionic surfactant and amphoteric surfactant;

preferably, the at least one surfactant is any one or more selected from the group consisting of ammonium dodecyl sulfate, N-dodecyl-$\beta$-D-maltoside, tridecyl-$\beta$-D-maltoside, sodium dodecyl sulfate, docusate sodium, cyclodextrin or derivatives thereof, chitosan or derivatives thereof, sodium polyoxyethylene alkyl sulfate, sodium laureth sulfate, sodium dioctyl sulfosuccinate, poloxamer, glyceryl monostearate, glycerol monolaurate, sorbitan monolaurate, sorbitan monostearate, polyoxyethylene sorbitan fatty acid esters, sorbitan fatty acid esters, polyoxyethylene monopalmitate, polyoxyethylene hydrogenated castor oil, $C_6$-$C_{12}$ fatty acid triglycerides and $C_8$-$C_{10}$

fatty acid triglycerides;

preferably, the at least one surfactant is selected from the group consisting of Tween, and preferably, the at least one surfactant is any one or more selected from the group consisting of Tween 20, Tween 40, Tween 60, Tween 65, Tween 80 and Tween 85.

11. The oral polypeptide composition according to any one of claims 1-10, wherein the weight ratio of the fish oil to the surfactant is 1: (0.2 to 15), preferably 1: (0.43 to 9), preferably 1: (0.43 to 5), preferably 1: (0.43 to 3), preferably 1: (0.43 to 2.33), and preferably 1: (0.43 to 1).

12. The oral polypeptide composition according to any one of claims 1-10, wherein the weight ratio of the fish oil to the surfactant is 1:1, or 1:2.33, or 1:0.43, or 1:9.

13. The oral polypeptide composition according to any one of claims 1-10, wherein the polypeptide is human insulin, the protease inhibitor is soybean trypsin inhibitor, the absorption enhancer is selected from ethylenediamine tetraacetic acid or salts thereof, and the surfactant is selected from Tween.

14. The oral polypeptide composition according to any one of claims 1-10, wherein the polypeptide is recombinant human insulin, the protease inhibitor is soybean trypsin inhibitor, the absorption enhancer is ethylenediamine tetraacetic acid or disodium ethylenediaminetetraacetate, and the surfactant is Tween 80.

15. A medicine comprising the oral polypeptide composition as claimed in claims 1 to 14, and preferably, further comprising a pharmaceutically acceptable auxiliary material.

16. The medicine according to claim 15, wherein the pharmaceutically acceptable auxiliary material includes a coating selected from the group consisting of gastric soluble coatings and/or enteric soluble coatings;

preferably, the coating is any one or more selected from the group consisting of hydroxypropyl cellulose, hydroxypropyl methylcellulose, acrylic resin, polyvinylpyrrolidone, cellulose acetate phthalate, hydroxypropyl methylcellulose phthalate and hydroxypropyl methylcellulose acetate succinate.

17. The medicine according to claim 16, wherein the pharmaceutically acceptable auxiliary material further includes any one or more of an excipient, a disintegrant, a binder, a flavoring agent, and a lubricant;

preferably, the excipient is any one or more selected from the group consisting of lactose, sorbitol, xylitol, mannitol, calcium sulfate, calcium carbonate, calcium hydrogen phosphate, microcrystalline cellulose, silicified microcrystalline cellulose, starch, pregelatinized starch, lactose starch complex, lactose cellulose complex, and mannitol starch complex;

preferably, the disintegrant is any one or more selected from the group consisting of cross-linked sodium carboxymethyl cellulose, cross-linked polyvinylpyrrolidone, sodium carboxymethyl starch, cross-linked sodium carboxymethyl starch, and low-substituted hydroxypropyl cellulose;

preferably, the binder is any one or more selected from the group consisting of starch slurry, gelatin solution, sucrose solution, methyl cellulose, ethyl cellulose, sodium carboxymethyl cellulose, hydroxypropyl methylcellulose, hydroxypropyl cellulose, and polyvinylpyrrolidone;

preferably, the lubricant is any one or more selected from the group consisting of sodium stearyl fumarate, magnesium lauryl sulfate, polyethylene glycol, magnesium dodecyl sulfate, sodium dodecyl sulfate, magnesium stearate, calcium stearate, micronized silica, and talc powder;

preferably, the flavoring agent is any one or more selected from the group consisting of mannitol, sorbitol, aspartame, stevioside, sucralose, aspartame, neotame, steviosin, sucralose and flavors.

18. The medicine according to any one of claims 15 to 17, wherein the medicine is prepared into any one or more dosage forms selected from the group consisting of a tablet, a pill, a capsule, an emulsion, a syrup or a suspension.

19. The medicine according to any one of claims 15 to 17, wherein the medicine is anhydrous.

20. A pharmaceutical composition comprising two or more different polypeptides, fish oil, a protease inhibitor, at least one absorption enhancer and at least one surfactant, wherein

the two or more different polypeptides are present in a total amount of 0.1 to 100 parts by weight;
the fish oil is present in an amount of 0.01 to 800 parts by weight;

the protease inhibitor is present in an amount of 0.1 to 210 parts by weight;

the absorption enhancer(s) is/are present in an amount of 0.1 to 300 parts by weight; and

the surfactant(s) is/are present in an amount of 1 to 800 parts by weight;

preferably,

the two or more different polypeptides are present in a total amount of 1 part by weight;

the fish oil is present in an amount of 0.01 to 800 parts by weight;

the protease inhibitor is present in an amount of 0.1 to 210 parts by weight;

the absorption enhancer(s) is/are present in an amount of 0.1 to 300 parts by weight; and

the surfactant(s) is/are present in an amount of 1 to 800 parts by weight;

preferably,

the two or more different polypeptides are present in the same formulation, or independently present in different formulations;

preferably, the two or more different polypeptides are any two or more selected from the group consisting of insulin or derivatives or analogues thereof, glucagon, glucagon-like peptide-1 receptor agonist, glucagon-like peptide-2 receptor agonist, calcitonin, growth hormone, somatostatin, thyrotropin-releasing hormone, parathyroid hormone, gonadotropin-releasing hormone, heparin, granulocyte colony-stimulating factor, prostaglandin, cyclosporin, interferon, vasopressin, vancomycin, erythrogenin, glutathione and thymosin;

more preferably, the two or more different polypeptides are any two or more selected from the group consisting of animal insulin, human insulin, recombinant human insulin, insulin derivatives, insulin analogues, glucagon, Exenatide, Benaglutide, Liraglutide, Loxenatide, Dulaglutide, Lixisenatide, Semaglutide, Albiglutide, Dulaglutide, Teduglutide, Lixisenatide, calcitonin, salmon calcitonin, growth hormone, Octreotide, thyrotropin-releasing hormone, parathyroid hormone fragment, Teriparatide, Abaloparatide, Leuprorelin, Protirelin, Gonadorelin, Goserelin, Buserelin, Sermorelin, Nafarelin, Histrelin, Triptorelin, Tesamorelin, Corticorelin, heparin, granulocyte colony-stimulating factor, prostaglandin, cyclosporin, interferon, vasopressin, vancomycin, erythrogenin, Lanreotide Acetate, Terlipressin, Antiangiotide, Nesiritide, Eptifibatide, Sifuvirtide, Atosiban, Ossotide, Muscular Amino Acids and Peptides and Nucleosides, glutathione, mannatide, Enfuvirtide, bradykinin, enkephalin, Nosiheptide, hirudin, Glatiramer acetate, aprotinin, Alarelin, pancreatic kininogenase, polymyxin, Serrapeptase, thymosin $\alpha$1, thymopentin and thymalfasin;

preferably, the two or more different polypeptides are any two or more selected from the group consisting of swine insulin, bovine insulin, human insulin, recombinant human insulin, Insulin Lispro, Insulin Aspart, isophane insulin, protamine zinc insulin, Insulin Glargine, Insulin Detemir, Insulin Degludec, glucagon, Exenatide, Benaglutide, Liraglutide, Loxenatide, Dulaglutide, Lixisenatide, Semaglutide, Albiglutide, Dulaglutide, Teduglutide, Lixisenatide, calcitonin, salmon calcitonin, recombinant growth hormone, Octreotide, thyrotropin-releasing hormone, parathyroid hormone fragment, Teriparatide, Abaloparatide, Leuprorelin, Protirelin, Gonadorelin, Goserelin, Buserelin, Sermorelin, Nafarelin, Histrelin, Triptorelin, Tesamorelin, Corticorelin, heparin, granulocyte colony-stimulating factor, prostaglandin, ciclosporin, recombinant human interferon $\alpha$2b, recombinant human interferon b, recombinant human interferon $\gamma$, vasopressin, vancomycin, erythrogenin, Lanreotide Acetate, Terlipressin, Antiangiotide, Nesiritide, Eptifibatide, Sifuvirtide, Atosiban, Ossotide, Muscular Amino Acids and Peptides and Nucleosides, glutathione, mannatide, Enfuvirtide, bradykinin, enkephalin, Nosiheptide, hirudin, Glatiramer acetate, aprotinin, Alarelin, pancreatic kininogenase, polymyxin, Serrapeptase, thymosin $\alpha$1, thymopentin and thymalfasin.

21. The pharmaceutical composition according to claim 20, wherein the pharmaceutical composition comprises two or more different polypeptides, fish oil, a protease inhibitor, at least one absorption enhancer and at least one surfactant, wherein

the two or more different polypeptides are present in a total mass fraction of 0.1% to 10.0%;

the fish oil is present in a mass fraction of 0.5% to 40.0%;

the protease inhibitor is present in a mass fraction of 5.0% to 28.0%;

the absorption enhancer(s) is/are present in a mass fraction of 8.0% to 42.0%; and

the surfactant(s) is/are present in a mass fraction of 10.0% to 55.0%;

preferably,

the two or more different polypeptides are present in a total mass fraction of 0.9% to 4.6%;

the fish oil is present in a mass fraction of 5.5% to 34.9%;

the protease inhibitor is present in a mass fraction of 10.0% to 22.6%;

the absorption enhancer(s) is/are present in a mass fraction of 12.9% to 36.1%; and

the surfactant(s) is/are present in a mass fraction of 15.0% to 49.6%;

preferably,

the two or more different polypeptides are present in a total mass fraction of 0.9% to 4.6%;
the fish oil is present in a mass fraction of 11.9% to 34.9%;
the protease inhibitor is present in a mass fraction of 10.0% to 22.6%;
the absorption enhancer(s) is/are present in a mass fraction of 12.9% to 36.1%; and
the surfactant(s) is/are present in a mass fraction of 15.0% to 34.5%;

preferably,

the two or more different polypeptides are present in a total mass fraction of 0.9% to 4.6%;
the fish oil is present in a mass fraction of 20.0% to 34.9%;
the protease inhibitor is present in a mass fraction of 10.0% to 17.2%;
the absorption enhancer(s) is/are present in a mass fraction of 12.9% to 35.6%; and
the surfactant(s) is/are present in a mass fraction of 15.0% to 34.5%;

preferably,

the two or more different polypeptides are present in a total mass fraction of 0.9% to 4.6%;
the fish oil is present in a mass fraction of 34.5% to 34.9%;
the protease inhibitor is present in a mass fraction of 10.0% to 17.2%;
the absorption enhancer(s) is/are present in a mass fraction of 12.9% to 35.6%; and
the surfactant(s) is/are present in a mass fraction of 15.0% to 34.5%.

22. The pharmaceutical composition according to claim 21, wherein the weight ratio of the fish oil to the surfactant is 1: (0.2 to 15), preferably 1: (0.43 to 9), preferably 1: (0.43 to 5), preferably 1: (0.43 to 3), preferably 1: (0.43 to 2.33), and preferably 1: (0.43 to 1).

23. The pharmaceutical composition according to claim 21, wherein the weight ratio of the fish oil to the surfactant is 1:1, or 1:2.33, or 1:0.43, or 1:9.

24. Use of the oral polypeptide composition according to any one of claims 1 to 14, the medicine according to any one of claims 15 to 19, or the pharmaceutical composition according to any one of claims 20 to 23 in the preparation of a drug for preventing or treating diabetes, obesity, reduced glucose tolerance, hepatic steatosis, hepatitis, liver fibrosis, cirrhosis and liver cancer, and for improving immunity.

25. Use of the oral polypeptide composition according to any one of claims 1 to 14, the medicine according to any one of claims 15 to 19, or the pharmaceutical composition according to any one of claims 20 to 23 in the preparation of a drug for preventing or treating a non-alcoholic fatty liver disease, wherein the non-alcoholic fatty liver disease is selected from the group consisting of simple fatty liver and/or non-alcoholic steatohepatitis.

26. Use of the oral polypeptide composition according to any one of claims 1 to 14, the medicine according to any one of claims 15 to 19, or the pharmaceutical composition according to any one of claims 20 to 23 in the preparation of a drug for preventing or treating non-alcoholic fatty liver disease complicated with diabetes, non-alcoholic fatty liver disease complicated with nephropathy, non-alcoholic fatty liver disease complicated with cardiovascular disease, non-alcoholic fatty liver disease complicated with obesity, and non-alcoholic fatty liver disease complicated with hyperlipidemia.

27. A method for treating or preventing a disease, wherein the method comprises orally administering a therapeutically effective amount of the oral polypeptide composition according to any one of claims 1 to 14, the medicine according to any one of claims 15 to 19, or the pharmaceutical composition according to any one of claims 20 to 23, to a subject in need thereof, and wherein the disease is selected from the group consisting of diabetes, obesity, reduced glucose tolerance, hepatic steatosis, hepatitis, liver fibrosis, cirrhosis, liver cancer, and immune diseases.

28. A method for treating or preventing a disease, wherein the method comprises orally administering a therapeutically effective amount of the oral polypeptide composition according to any one of claims 1 to 14, the medicine according to any one of claims 15 to 19, or the pharmaceutical composition according to any one of claims 20 to 23, to a subject in need thereof, and wherein the disease is a non-alcoholic fatty liver disease.

29. A method for treating or preventing a disease, wherein the method comprises orally administering a therapeutically effective amount of the oral polypeptide composition according to any one of claims 1 to 14, the medicine according to any one of claims 15 to 19, or the pharmaceutical composition according to any one of claims 20 to 23, to a subject in need thereof, and wherein the disease is selected from the group consisting of non-alcoholic fatty liver disease complicated with diabetes, non-alcoholic fatty liver disease complicated with nephropathy, non-alcoholic fatty liver disease complicated with cardiovascular disease, non-alcoholic fatty liver disease complicated with obesity, and non-alcoholic fatty liver disease complicated with hyperlipidemia.

30. The method for treating or preventing a disease according to any one of claims 27 to 29, comprising administering orally to the subject the oral polypeptide composition according to any one of claims 1 to 14 at a single dose of 8 to 32 mg of polypeptide, 1 to 3 times a day, 1 to 3 capsules per time; or, administering orally to the subject the medicine according to any one of claims 15 to 19 at a single dose of 8 to 32 mg of polypeptide, 1 to 3 times a day, 1 to 3 capsules per time; or, administering orally to the subject the pharmaceutical composition according to any one of claims 20 to 23 at a single dose of 8 to 32 mg of polypeptide, 1 to 3 times a day, 1 to 3 capsules per time.

31. The method for treating or preventing a disease according to any one of claims 27 to 29, comprising administering orally to the subject the oral polypeptide composition according to any one of claims 1 to 14 at a single dose of 8 mg, 16 mg or 32 mg of polypeptide, 1, 2 or 3 times a day, 1, 2 or 3 capsules per time; or, administering orally to the subject the medicine according to any one of claims 15 to 19 at a single dose of 8 mg, 16 mg or 32 mg of polypeptide, 1, 2 or 3 times a day, 1, 2 or 3 capsules per time; or, administering orally to the subject the pharmaceutical composition according to any one of claims 20 to 23 at a single dose of 8 mg, 16 mg or 32 mg of polypeptide, 1, 2 or 3 times a day, 1, 2 or 3 capsules per time.

# EP 4 631 514 A1

## INTERNATIONAL SEARCH REPORT

International application No.

**PCT/CN2023/137373**

### A. CLASSIFICATION OF SUBJECT MATTER

A61K38/00(2006.01)i; A61K38/28(2006.01)i; A61K45/00 (2006.01)i; A61K47/32(2006.01)i; A61K47/26(2006.01)i; A61K9/00(2006.01)i; A61P3/10(2006.01)i

According to International Patent Classification (IPC) or to both national classification and IPC

### B. FIELDS SEARCHED

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNTXT, ENTXT, DWPI, CNKI, CAPLUS, REGISTRY 合肥天汇生物科技有限公司, 张磊, 徐平, 陈书昊, 吕荟, 庞迪, 樊艾嘉, 多肽, 胰岛素, 鱼油, 蛋白酶抑制剂, 大豆胰蛋白酶抑制剂, 乙二胺四乙酸二钠, 表面活性剂, 吐温, polypeptide, insulin, fish oil, DHA, EPA, protease inhibitor, soybean trypsin inhibitor, disodium ethylenediaminetetraacetate, edta, surfactant, tween

### C. DOCUMENTS CONSIDERED TO BE RELEVANT

| Category* | Citation of document, with indication, where appropriate, of the relevant passages | Relevant to claim No. |
|---|---|---|
| X | CN 101547702 A (ORAMED PHARMACEUTICALS, INC.) 30 September 2009 (2009-09-30) <br>     description, page 19, paragraphs 1-3, page 21, paragraph 4, and page 17, paragraph 3 | 1-31 |
| X | CN 113230386 A (HEFEI TIANHUI HATCHING TECHNOLOGY CO., LTD.) 10 August 2021 (2021-08-10) <br>     description, paragraphs [0052]-[0062] and [0032]-[0033] | 1-31 |
| A | CN 113117050 A (HEFEI TIANHUI HATCHING TECHNOLOGY CO., LTD.) 16 July 2021 (2021-07-16) <br>     claim 1 | 1-31 |
| A | CN 115400205 A (SHANGHAI HAIZE BIOLOGICAL TECHNOLOGY CO., LTD.; HEFEI TIANHUI ANCHAO BIOTECHNOLOGY CO., LTD.) 29 November 2022 (2022-11-29) <br>     claim 1 | 1-31 |
| A | CN 109498559 A (FUDAN UNIVERSITY) 22 March 2019 (2019-03-22) <br>     claim 1 | 1-31 |

☐ Further documents are listed in the continuation of Box C.    ☑ See patent family annex.

| | |
|---|---|
| *   Special categories of cited documents: <br> "A"   document defining the general state of the art which is not considered to be of particular relevance <br> "D"   document cited by the applicant in the international application <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **20 March 2024** | **31 March 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/137373**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **27-31**
   because they relate to subject matter not required to be searched by this Authority, namely:

   These claims fall within methods for treatment of diseases (PCT Rule 39.1(iv)); however, a search is still carried out with regard to the corresponding pharmaceutical use of the compound.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/137373**

| Patent document cited in search report | | | Publication date (day/month/year) | Patent family member(s) | | | Publication date (day/month/year) |
|---|---|---|---|---|---|---|---|
| CN | 101547702 | A | 30 September 2009 | ES | 2462117 | T3 | 22 May 2014 |
| | | | | JP | 2009507067 | A | 19 February 2009 |
| | | | | JP | 5222727 | B2 | 26 June 2013 |
| | | | | EP | 1933862 | A2 | 25 June 2008 |
| | | | | EP | 1933862 | A4 | 28 December 2011 |
| | | | | EP | 1933862 | B1 | 26 March 2014 |
| | | | | WO | 2007029238 | A2 | 15 March 2007 |
| | | | | WO | 2007029238 | A3 | 30 April 2009 |
| | | | | US | 2007087957 | A1 | 19 April 2007 |
| | | | | US | 9259456 | B2 | 16 February 2016 |
| | | | | HK | 1119951 | A1 | 20 March 2009 |
| | | | | CA | 2621577 | A1 | 15 March 2007 |
| | | | | CA | 2621577 | C | 24 December 2013 |
| | | | | EP | 2722054 | A1 | 23 April 2014 |
| | | | | EP | 2722054 | B1 | 21 March 2018 |
| | | | | AU | 2006288703 | A1 | 15 March 2007 |
| | | | | AU | 2006288703 | B2 | 22 September 2011 |
| | | | | ES | 2670856 | T3 | 01 June 2018 |
| | | | | US | 2022160839 | A1 | 26 May 2022 |
| | | | | US | 2016206703 | A1 | 21 July 2016 |
| CN | 113230386 | A | 10 August 2021 | None | | | |
| CN | 113117050 | A | 16 July 2021 | None | | | |
| CN | 115400205 | A | 29 November 2022 | None | | | |
| CN | 109498559 | A | 22 March 2019 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Chinese Pharmacopoeia. 2020, vol. IV